# EUROPEAN PATENT SPECIFICATION

(11) **EP 2 646 792 B1**
(45) Date of publication and mention of the grant of the patent: **10.07.2019**
(21) Application number: 11788311.6
(22) Date of filing: 17.11.2011
(51) Int. Cl.: G01N 1/08, G01N 1/42, G01N 1/28

(54) **APPARATUS AND METHODS FOR ALIQUOTTING FROZEN SAMPLES**
VORRICHTUNG UND VERFAHREN ZUR ALIQUOTIERUNG GEFRORENER PROBEN
APPAREIL ET PROCÉDÉS PERMETTANT D'ALIQUOTER DES ÉCHANTILLONS CONGELÉS

(30) Priority: 01.12.2010 US 418688 P
(43) Date of publication of application: 09.10.2013
(73) Proprietor: President and Fellows of Harvard College, Cambridge, MA 02138 (US); Charles Stark Draper Laboratory, Inc., Cambridge, MA 02139 (US)
(72) Inventor: LARSON, Dale, N., Cambridge, Massachusetts 02139 (US); BELLIO, Stephen, L., Cambridge, Massachusetts 02139 (US); SLUSARZ, John, Cambridge, Massachusetts 02138 (US)
(74) Representative: Potter Clarkson
(86) International application number: PCT/US2011/061214
(87) International publication number: WO 2012/074771

(56) References cited:
- US-A1- 2002 177 119
- US-A1- 2009 019 877
- Serena Donnelly Webb ET AL: "Freezing Biopharmaceuticals Using. Common Techniques - and the. Magnitude of Bulk-Scale. Freeze-Concentration", , 1 May 2002 (2002-05-01), pages 1-8, XP055017070, Retrieved from the Internet: URL:http://www.sartorius-stedim.fr/fileadm in/sartorius_pdf/stedim/intgrated_bio/BP58 89e.pdf [retrieved on 2012-01-20]
- U. T. LASHMAR ET AL: "Bulk Freeze-Thawing of Macromolecules: Effects of Cryoconcentration on Their Formulation and Stability", BIOPROCESS INTERNATIONAL, vol. 5, no. 6, 1 June 2007 (2007-06-01), pages 44-55, XP055017062,
- MAITY H ET AL: "Mapping of solution components, pH changes, protein stability and the elimination of protein precipitation during freeze-thawing of fibroblast growth factor 20", INTERNATIONAL JOURNAL OF PHARMACEUTICS, ELSEVIER BV, NL, vol. 378, no. 1-2, 13 August 2009 (2009-08-13), pages 122-135, XP026348606, ISSN: 0378-5173 [retrieved on 2009-06-06]

## Description

### FIELD OF INVENTION

The present invention relates generally to methods for aliquotting frozen samples, and more particularly to methods for efficiently taking multiple frozen sample cores from a single frozen biological sample to provide a plurality of aliquots that can be analyzed separately without thawing the sample and while minimizing degradation of the sample, protecting the integrity of the sample, and prolonging its usable life.

### BACKGROUND

Biological samples include any samples which are of animal (including human), plant, protozoal, fungal, bacterial, viral, or other biological origin. For example, biological samples include, but are not limited to, organisms and/or biological fluids isolated from or excreted by an organism such as plasma, serum, urine, whole blood, cord blood, other blood-based derivatives, cerebral spinal fluid, mucus (from respiratory tract, cervical), ascites, saliva, amniotic fluid, seminal fluid, tears, sweat, any fluids from plants (including sap); cells (e.g., animal, plant, protozoal, fungal, or bacterial cells, including buffy coat cells; cell lysates, homogenates, or suspensions; microsomes; cellular organelles (e.g., mitochondria); nucleic acids (e.g., RNA, DNA), including chromosomal DNA, mitochondrial DNA, and plasmids (e.g., seed plasmids); small molecule compounds in suspension or solution (e.g. small molecule compounds in DMSO); and other fluid-based biological samples. Biological samples may also include plants, portions of plants (e.g., seeds) and tissues (e.g., muscle, fat, skin, etc.).

Bio-banks typically cryopreserve these valuable samples (e.g., in freezers at -80 degrees centigrade using liquid Nitrogen or the vapor phase above liquid Nitrogen) to preserve the biochemical composition and integrity of the frozen sample as close as possible to the in vivo state to facilitate accurate, reproducible analyses of the samples.

From time to time, it may be desirable to run one or more tests on a sample that has been frozen. For example, a researcher may want to perform tests on a set of samples having certain characteristics. A particular sample may contain enough material to support a number of different tests. In order to conserve resources, smaller samples known as aliquots are commonly taken from larger cryopreserved samples for use in one or more tests so the remainder of the cryopreserved sample will be available for one or more different future tests.

Biobanks have adopted a couple of different ways to address this. One option is to freeze a sample in large volume, thaw it when aliquots are requested and then refreeze any remainder for storage in the cryopreserved state until future aliquots are needed. This option makes efficient use of freezer space; yet this efficiency comes at the cost of sample quality. Repeated freeze/thaw cycles can degrade critical biological molecules (e.g., RNA) and damage biomarkers, either of which could compromise the results of any study using data obtained from the damaged samples.

Another option is to freeze a sample in large volume, thaw it when an aliquot is requested, subdivide the remainder of the sample to make additional aliquots for future tests and then refreeze these smaller volume aliquots to cryopreserve each aliquot separately until needed for a future test. This approach limits the number of freeze/thaw cycles to which a sample is exposed, but there is added expense associated with labor, the larger volume of freezer space, and a larger inventory of containers required to maintain the cryopreserved aliquots. Moreover, the aliquots can be degraded or damaged by even a limited number freeze/thaw cycles. Yet another approach is to divide a large volume sample into smaller volume aliquots before freezing. This approach enables the freeze thaw cycles to be limited to only one, yet there are disadvantages associated with costs of labor, freezer space, and container inventory with this approach.

U.S. pre-grant publication No. 20090019877, discloses a system for extracting frozen sample cores from a single frozen biological sample without thawing the sample. The system uses a drill including a hollow bit coring needle to take a frozen core sample from the original sample without thawing the sample. The frozen sample core obtained by the drill is used as the aliquot for the test. After the frozen core is removed, the remainder of the sample is returned to the freezer until another aliquot from the sample is needed for a future test. The present inventors have made various improvements on the system disclosed in the '877 publication, as will be described in detail below. SERENA DONNELLY WEBB ET AL: "Freezing Biopharmaceuticals Using. Common Techniques - and the. Magnitude of Bulk-Scale. Freeze-Concentration", 1 May 2002 (2002-05-01), pages 1 - 8 provides a review of various known processes for freeze drug products for storage.

### SUMMARY

According to the present invention there is provided a method of obtaining an aliquot of a frozen sample contained in a container (c) in accordance with Claims 1 and 8.

Other objects and features will in part be apparent and in part pointed out hereinafter.

### BRIEF DESCRIPTION OF DRAWINGS

FIG. 1 is a perspective of one example an aliquotting system that can be used to perform the methods of the present invention;
FIG. 2 is a perspective of the system with the outer enclosure removed;
FIG. 3 is an exploded perspective of the system as illustrated in Fig. 2;
FIG. 4 is an enlarged perspective similar to Fig. 2 but with a robot arm removed and portions of a cover broken away to reveal a platform for supporting sample containers;
FIG. 5 is a front elevation of the system as illustrated in Fig. 4 with a portion of the cover broken away;
FIG. 6 is a top plan view of the system as illustrated in Figs. 4 and 5 with a different portion of the cover broken away;
FIG. 7 is a section of the system taken in a plane including line 7--7 on Fig. 6;
FIG. 8 is a perspective of the system sectioned as illustrated in Fig. 7 and with the cover removed;

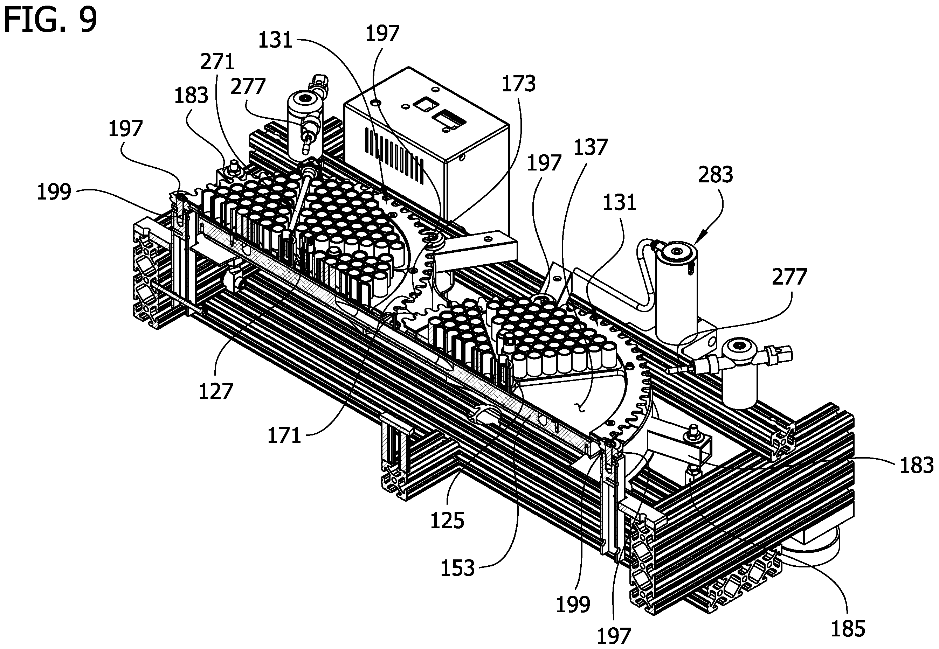

FIG. 9 is a perspective of the system sectioned in a plane including line 9--9 on Fig. 6;
FIGS. 10, 10A, 11, and 11A are enlarged sections of a portion of the system taken in a plane including line 10--10 on Fig.6, illustrating operation of a clamping system for retaining containers;
FIG. 12 is an enlarged section taken in a plane including line 12--12 on Fig. 6 showing a portion of the frame of the system;
FIG. 13 is a perspective of the robot arm of the system;
FIG. 14 is a section of the robotic arm taken in a plane including line 14--14 on Fig. 13;
FIG. 15 is an exploded perspective of a portion of the robot arm;
FIGS. 16A and 16B illustrate a sequence in which a plunger in the robot arm ejects a frozen sample core from a coring bit;
FIGS. 17A-17F illustrate operation of one example of a cleaning system adapted to clean the plunger and coring bit;
FIGS. 18A and 18B are schematic diagrams illustrating a frozen sample from which multiple frozen sample cores have already been taken being inspected by a sample inspection device;
FIG. 18C is a graph illustrating the output from a sensor adapted to identify locations within a frozen sample from which aliquots have previously been take;
FIG. 18D is a schematic diagram illustrating use of a time-of-flight based distance sensor to inspect a sample;
FIG. 19 is an enlarged perspective of the tip of the coring bit;
FIG. 20 is a perspective of a removable tray for holding containers on the system; and
FIGS. 21 and 22 are perspectives of a toggle mechanism for selectively clamping and releasing containers on a platform of the system.

Corresponding reference characters indicate corresponding parts throughout the drawings.

### DETAILED DESCRIPTION

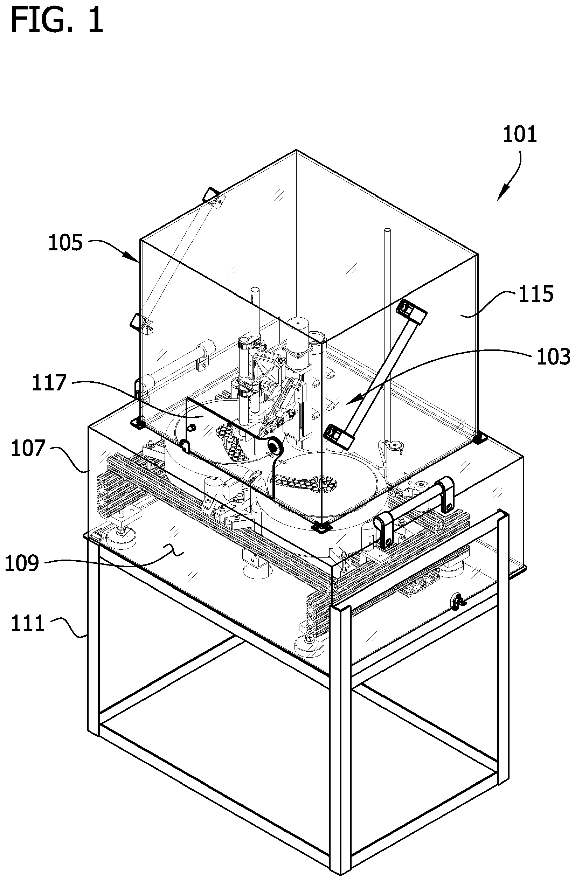

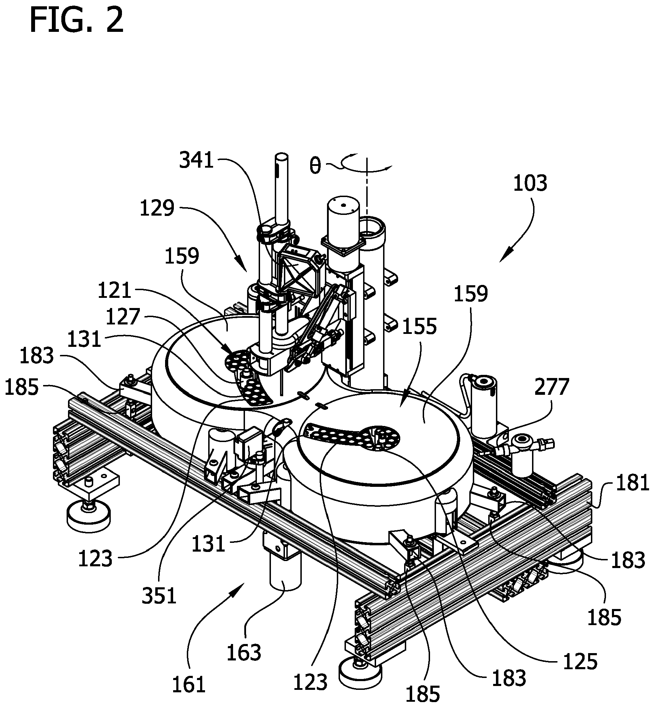

Referring now to the drawings, first to Figs. 1 and 2, one example of an automated frozen sample aliquotting system, generally designated 101, includes a robot 103 adapted to automatically take a plurality of frozen aliquots (e.g., in the form of frozen sample cores) from a plurality of different frozen samples. The samples are suitably frozen biological samples.

Biological samples include any samples which are of animal (including human), plant, protozoal, fungal, bacterial, viral, or other biological origin. For example, biological samples include, but are not limited to, organisms and/or biological fluids isolated from or excreted by an organism -- such as plasma, serum, urine, whole blood, cord blood, other blood-based derivatives, cerebral spinal fluid, mucus (from respiratory tract, cervical), ascites, saliva, amniotic fluid, seminal fluid, tears, sweat, any fluids from plants (including sap), cells (e.g., animal, plant, protozoal, fungal, or bacterial cells, including buffy coat cells; cell lysates, homogenates, or suspensions; microsomes; cellular organelles (e.g., mitochondria); nucleic acids (e.g., RNA, DNA), including chromosomal DNA, mitochondrial DNA, and plasmids (e.g., seed plasmids), small molecule compounds in suspension or solution (e.g. small molecule compounds in DMSO), and other fluid-based biological samples. Biological samples may also include plants, portions of plants (e.g., seeds), and tissues (e.g., muscle, fat, organs, skin, etc.).

The robot 103 includes or is connected to a processor (not shown), such as a computer, that controls operation of the robot. The robot 103 is positioned in an enclosure 105 which protects the robot and also protects an operator from any sharp objects, aerosols, or spray that might be associated with operation of the robot. In the case of frozen biological samples, which might contain pathogens (e.g., blood-borne pathogens), the enclosure 105 also limits exposure of the operator to potential pathogens. The ability to limit release of sample materials into the environment can be important when the aliquotting system is used to take aliquots from frozen biological samples or other potentially hazardous materials.

Various different enclosures can be used within the scope of the invention. For example, the enclosure 105 in Fig. 1 includes a base 107 enclosing a lower chamber 109 in which the base of the robot 103 is contained. As illustrated, the base 107 of the enclosure 105 is supported by a stand 111, e.g., a cart having wheels (not shown), so the robot 103 is supported above the floor at a comfortable working height to facilitate manual loading and unloading of samples and aliquots from the robot and other manual work. The cart 111 also makes it easy to move the robot from one location to another. The base 107 of the enclosure 105 could instead be positioned on a table or work bench. It is also understood the base of the robot 103 does not need to be supported at any particular elevation within the broad scope of the invention.

The enclosure 105 also includes a removable cover 115 atop the enclosure base 107. The cover 115 can be removed to access the robot 103 for maintenance or repair as may be needed from time to time. The enclosure 105 also includes a door 117 on the front of the cover 115 that can be opened to load and unload the frozen sample vials and frozen aliquot vials from the system and/or perform limited maintenance or repairs on the system 101 without removing the cover. The cover 115 also helps insulate the frozen samples from thermal variables in the room containing the robot 103. It is contemplated that the enclosure 105 can be omitted from the system, such as if the circumstances permit operation without any enclosure or the system is to be placed in a separate fume or biological hood or other device that sufficiently addresses any concerns about release of materials from the samples into the environment.

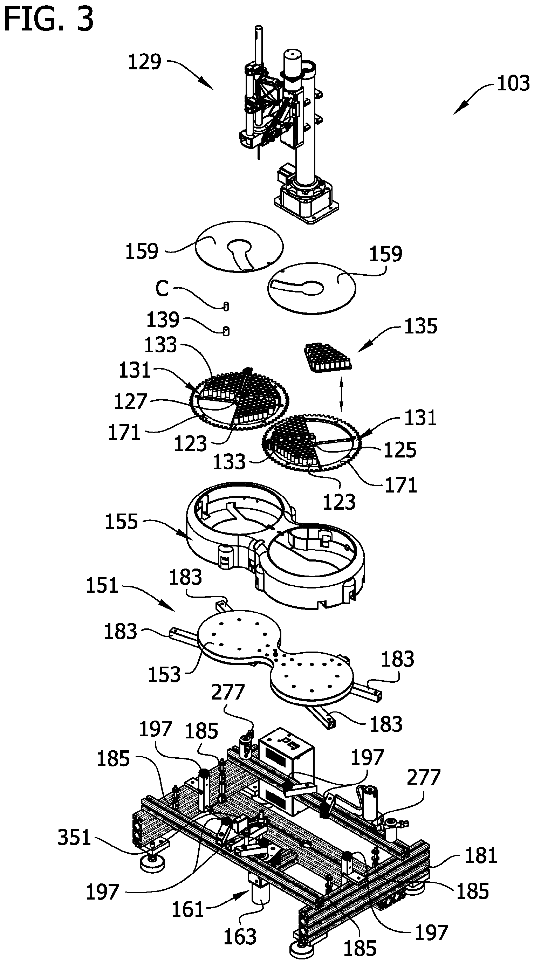

Referring to Figs. 2 and 3, the robot 103 includes a high-precision automated positioning system 121 adapted to move multiple sample-containing and aliquot-receiving containers C (e.g., vials) between temporary storage locations 123 and a sample-coring station 125 or aliquot receiving station 127, respectively. The high precision positioning system 121 facilitates obtaining aliquots in the form of frozen sample cores from precise locations within the sample containers. For example, the positioning system 121 is suitably capable of maintaining actual positioning that is within a few thousandths of an inch (e.g., about 4 thousandths of an inch) of targeted positioning, while conducting operations in a cold environment adapted to minimize undesirable thermally-induced changes to frozen biological samples. This can facilitate taking the frozen sample cores from locations that are selected to optimize the quality of the aliquot (e.g., as will be described in greater detail later) and/or maximizing the number of aliquots that can be taken from a single sample container. Although the ability to maintain precise positioning can be advantageous, it is not necessary within the broad scope of the invention.

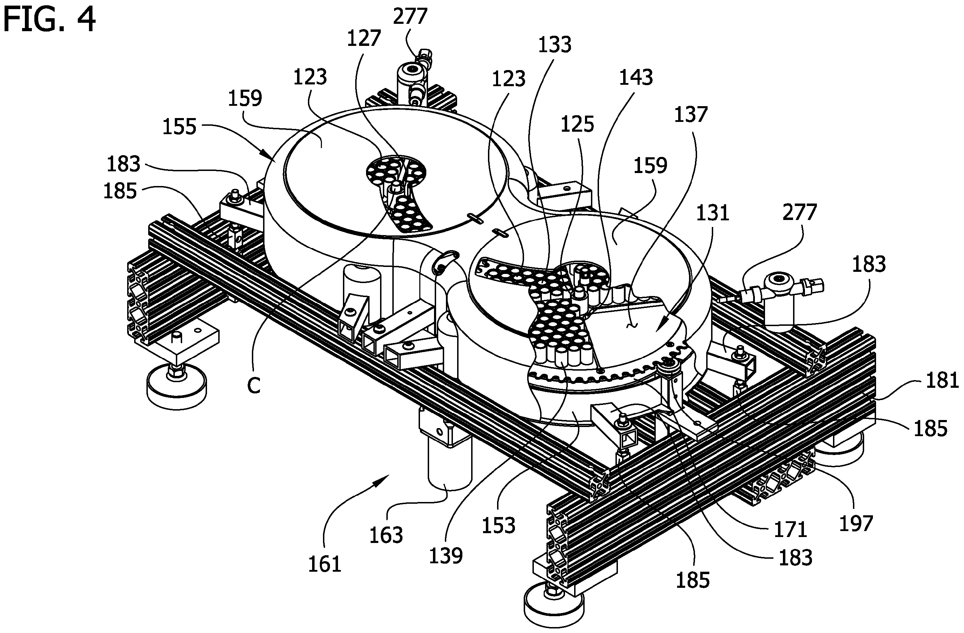

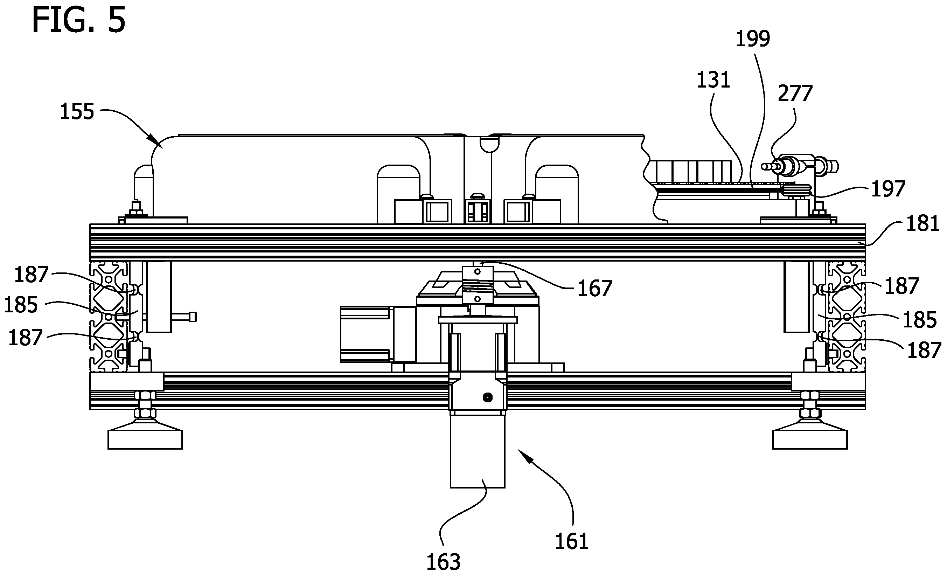

Although it is possible to use an x, y, z, Cartesian coordinate positioning system within the broad scope of the invention, the positioning system 121 in the illustrated example is a θ, θ', z positioning system. The positioning system 121 suitably includes one or more moveable platforms 131 for supporting the containers and a robotic arm 129 (the details of which will be described later) adapted to pick up samples off the platform(s), move the samples relative to the platform(s), and then place the samples down at a different position relative to the platform(s). As illustrated in Figs. 3-6, for example, the positioning system 121 includes two rotatably-mounted platforms 131 (e.g., turntables) that support the containers c and a servo-controlled drive system 161 adapted to control movement of the turntables. The turntables 131 suitably support a plurality of receptacles 133 in which the containers C can be received to hold the containers at fixed positions relative to the respective turntable. The sample coring station 125 is suitably a receptacle 133 at the center of one of the turntables 131 and the aliquot receiving station 127 is suitably a receptacle at the center of the other turntable.

In the illustrated example, the turntables 131 are adapted to support a plurality of removable trays 135, each of which is capable of supporting a plurality of containers C. For example, the upper surfaces of the turntables 131 may include recesses 137 configured to receive the trays 135 and hold them in position on the turntables. Each receptacle 133 in the illustrated example is defined by an upright peripheral (cylindrical) sidewall supported by one of the trays 135. For example, open-ended cylindrical sleeves 139 can be inserted into wells 141 on the upper surfaces of the trays 135 to form the receptacles 133. Similar sleeves 139 are inserted into wells 143 at the center of the turntables 131 to form receptacles 133 at the sample coring and aliquot receiving stations 125, 127. The sleeves 139 are suitably made of a solid thermally conductive material such as metal and have a relatively high thermal mass so frozen samples or frozen aliquots inside the sleeves are thermally protected by the sleeve.

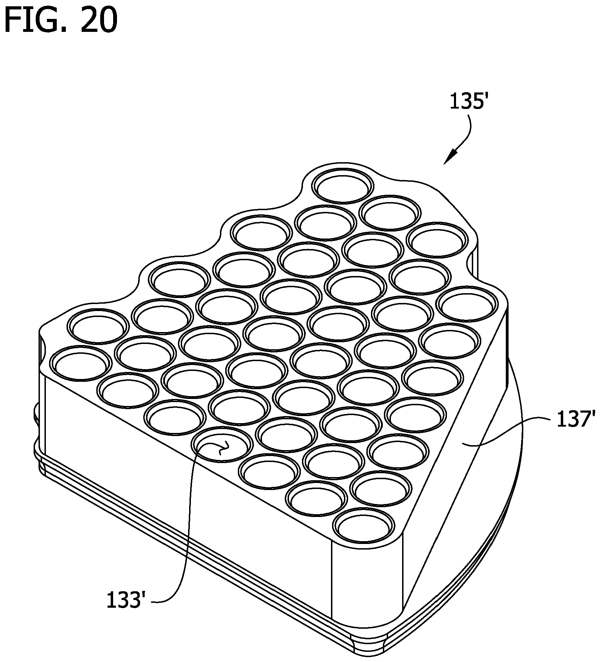

Another example of a tray 135' is illustrated in Fig. 20. This tray 135' is a one-piece tray or tray insert in which relatively deep receptacles 133' are formed in a solid one-piece body 137' so the body extends up along the sides of a sample to provide thermal protection for the sample when the sample is received in one of the receptacles.

The removable trays 135, 135' facilitate loading and unloading containers from the turntables 131 because an entire tray and the containers thereon can be loaded or unloaded together in a single step. The receptacles 133, 133' in the illustrated example are circular in shape. It is also understood that there are various other ways to adapt a platform for supporting a plurality of containers without using any trays or receptacles within the broad scope of the invention.

The drive system 161 for the turntables 131 suitably includes a single precision motion control device 163 (e.g.,servo-motor) adapted to control movement of both turntables 131. As used herein the term "precision motion control device" refers to a mechanical drive system adapted to track a drive output, such as position or velocity, and ensure that a desired drive output is attained. Precision motion control devices include servo-motors and servo-mechanisms, which refer to motors or drive mechanisms having control systems that use feedback to provide precise control (e.g., positional control) of the motor/mechanism and/or one or more structures driven by the device. For example, servo-motors and servo-mechanisms can include control systems that use feedback from one or more position-indicating sensors (not shown) to achieve precise control of the output. The term precision motion control also includes stepper motors, which have motor control systems that track output of the motor by counting the number of steps through which the rotor has been rotated.

One feature of the system 101 is that it uses a relatively low number of precision motion control devices to perform a large number of varied tasks, as will be described below. Because precision motion control devices are relatively expensive to build or purchase and because significant maintenance is required to keep a precision motion control device in good working order, the ability to limit the number of precision motion control devices in the system 101 provides advantages. Using a precision motion control device (e.g., a single servo-mechanism 136) to control movement of both turntables is one aspect of this feature of the system 101. Others that may also be present will be described later.

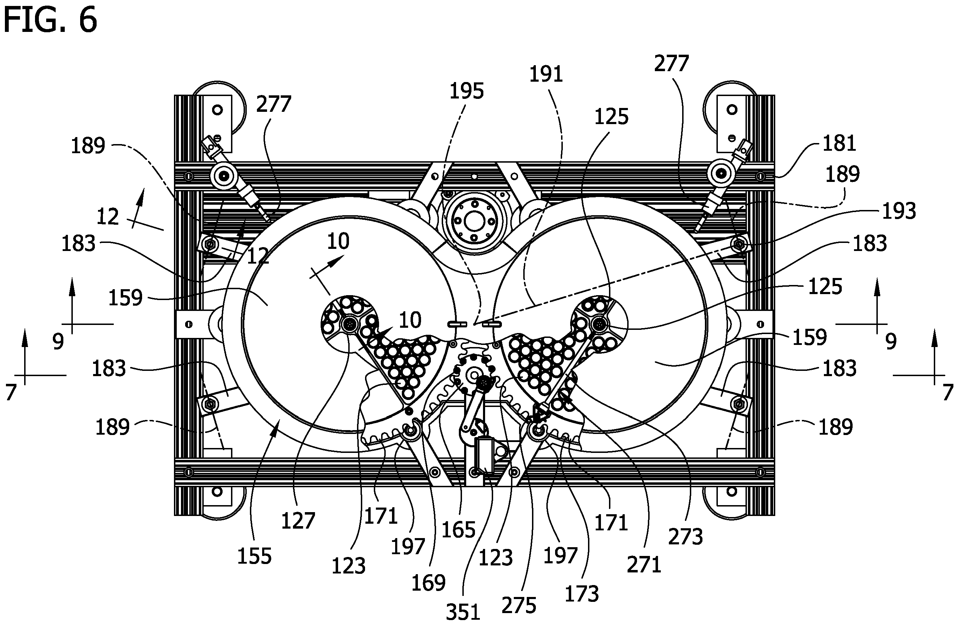

Referring to Figs. 6-8, the turntable drive motor 163 is mounted under the turntables 131 on a frame 181. The frame 181 also supports the turntables 131 in generally side-by-side relation to one another. For instance, the frame 181 suitably supports a plurality of rollers 197 positioned to engage a radially-outward extending lip 199 on each of the turntables 131 to support the turntables for rotation relative to the frame. The lip 199 is received in a notch (e.g., V-shaped notch) in each of the rollers 197 so the rollers hold the turntable lip at a fixed elevation. As illustrated in Fig. 3, there are three rollers 197 for each turntable 131.

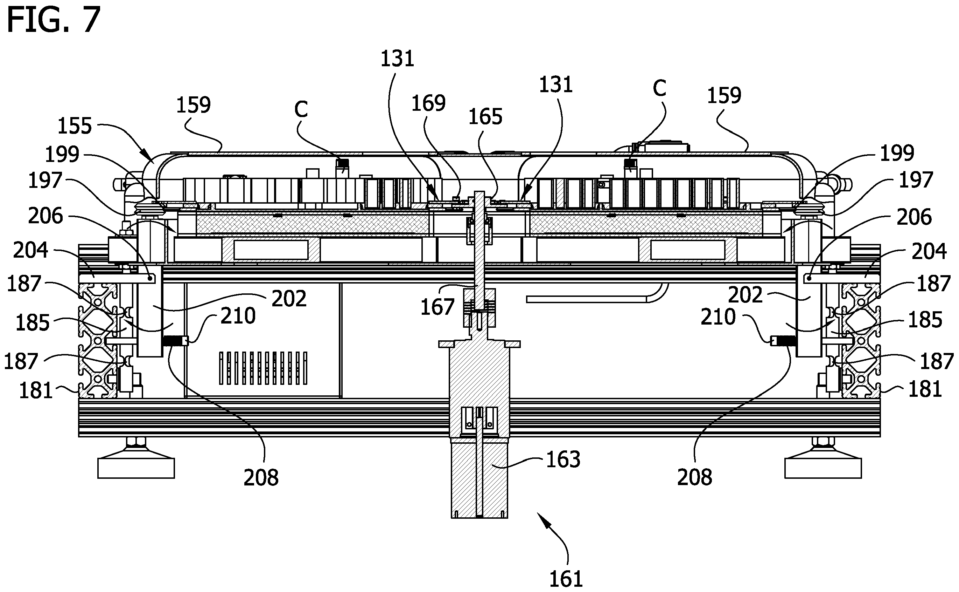

One or more of the rollers 197 for each turntable is suitably moveable in the radial direction and biased (e.g., by a spring) to move radially inward toward the center of the turntable to ensure the rollers maintain engagement with the turntable lip 199. As illustrated in Fig. 7, for example, the rollers 197 mounted on the far left and right sides of the frame 181 are mounted on a roller support arm 202, which has a generally upright orientation in the illustrated example. The support arm 202 is moveable relative to the frame and biased to move the roller radially inward. In the illustrated example, the support arm 202 is secured to the frame 181 by a bracket 204 that allows pivoting movement of the arm about a pivot axis 206. A spring 208 or other biasing member is positioned to bias the support arm 202 to pivot in the direction in which the roller moves toward the center of the turntable, as indicated by arrows in Fig. 7. For example, the spring 208 is suitably mounted under the pivot axis 206 and compressed between a side of the support arm 202 and the head of a bolt 210 or other suitable retaining structure fixed to the frame 181. The bolt 210 is suitably adjustable to increase or decrease preload applied to the spring 208. Because one of the rollers 197 for each turntable can move in the radial direction, the rollers 197 are able to accommodate thermal expansion and/or contraction of the turntables 131, while the bias from the preloaded spring 208 ensures the rollers remain snuggly engaged with the turntables.

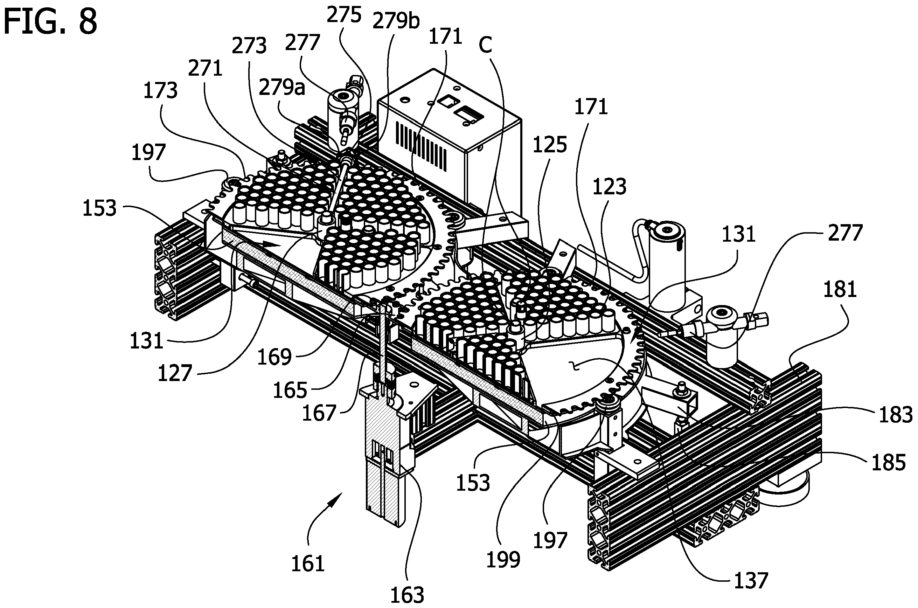

Referring again to Figs. 6-8, the motor 163 is operably connected (e.g., by a drive shaft 167 as illustrated in Fig. 8) to a wheel 165 so motor is operable to rotate the wheel. The wheel 165 is adjacent the turntables 131 (e.g., generally between the turntables) and is operably connected to each of the turntables 131 so rotation of the wheel by the motor 163 drives rotation of both turntables. For example, the wheel 165 is suitably positioned to simultaneously engage the opposing edges of the turntables 131 so the turntables are rotated at the same time.

In particular, the wheel 165 in the illustrated example supports a plurality of rotatable pegs 169 and the turntables 131 have teeth 171 that are enmeshed with the pegs so the turntables are turned by the pegs. This results in the turntables 131 being rotated in unison (i.e., at the same time, in the same direction, and at the same speed), but it is understood the turntables are not required to rotate in unison within the broad scope of the invention. The pegs 169 are suitably substantially cylindrical in cross sectional shape and the spaces between the teeth 171 are shaped so they substantially conform to the pegs, as illustrated. The pegs 169 can be made from or include roller bearings, ball bearings, or other bearings (not shown) that allow the pegs/bearings to rotate relative to the wheel 165 (e.g., on vertical axes when oriented as illustrated) while engaged with either of the turntables 131. The wheel 165 and pegs 169 result in a low-friction, low-backlash, positive drive connection between the motor 163 and the turntables 131, which facilitates very precise positioning of the turntables and the containers C thereon by the positioning system 121.

The system 101 includes a temperature control system 151 (See Fig. 3) operable to maintain the frozen samples and aliquots at a desired temperature (e.g., at cryogenic temperatures suitably in the range of about 0 degrees centigrade and about -180 degrees centigrade, more suitably in the range of about - 40 degrees centigrade and about -180 degrees centigrade, more suitably in the range of about -40 degrees centigrade to about - 80 degrees centigrade) while they are on the turntables 131 to limit sample degradation and protect sample and aliquot integrity. It will usually be desirable to operate the temperature control system 151 so the temperature is lower than about - 40 degrees centigrade. The temperature control system 151 suitably is programmed to accept input from an operator for setting or adjusting the desired temperature.

As illustrated, the temperature control system 151 includes a temperature control block 153 positioned under the turntables 131 and an enclosure 155 partially enclosing the temperature control block, along with the turntables 131 and the containers thereon. The enclosure 155 includes a pair of removable covers 159. Each of the turntables 131 is covered by one of the covers 159. Various different temperature control blocks can be used to control the temperature of the frozen samples within the scope of the invention. For example, the temperature control block 153 can include a reservoir or passages (not shown) for containing a cooling fluid, such as liquid nitrogen or ethanol. By keeping the samples very cold, the temperature control system 151 helps limit (and ideally prevent) melting of the frozen samples during the aliquotting process. The temperature control system 151 also limits frost formation on the frozen samples. It is undesirable for frost to form on the samples because water in the frost can dilute the sample material and thereby alter the results of any quantitative analysis performed on the sample. Although the temperature control system 151 in the illustrated example provides only cooling, it is contemplated that the temperature control system may heat frozen samples within the scope of the invention. For instance, it may be desirable in some cases to warm the frozen samples slightly above their cryogenic storage temperature to make the frozen samples less susceptible to cracking or other physical damage during the aliquotting process.

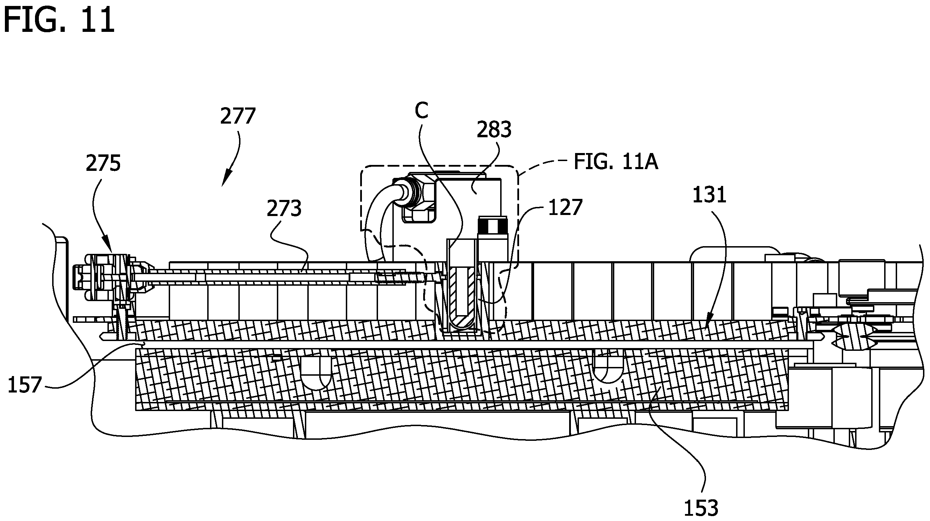

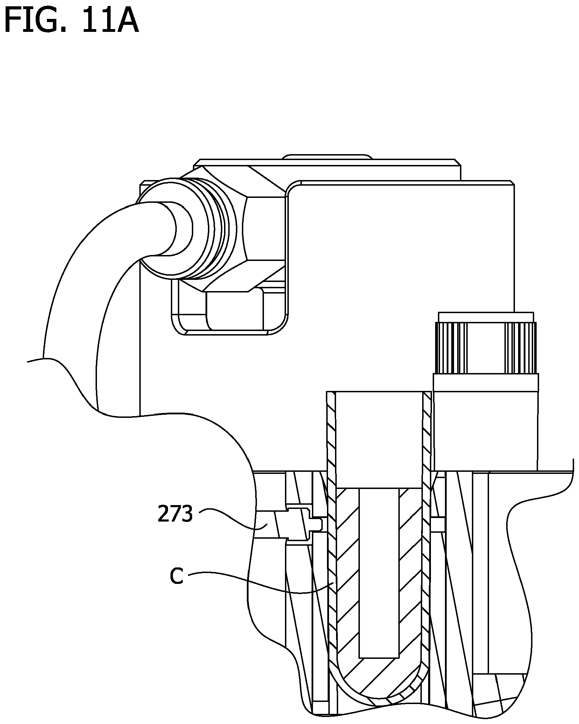

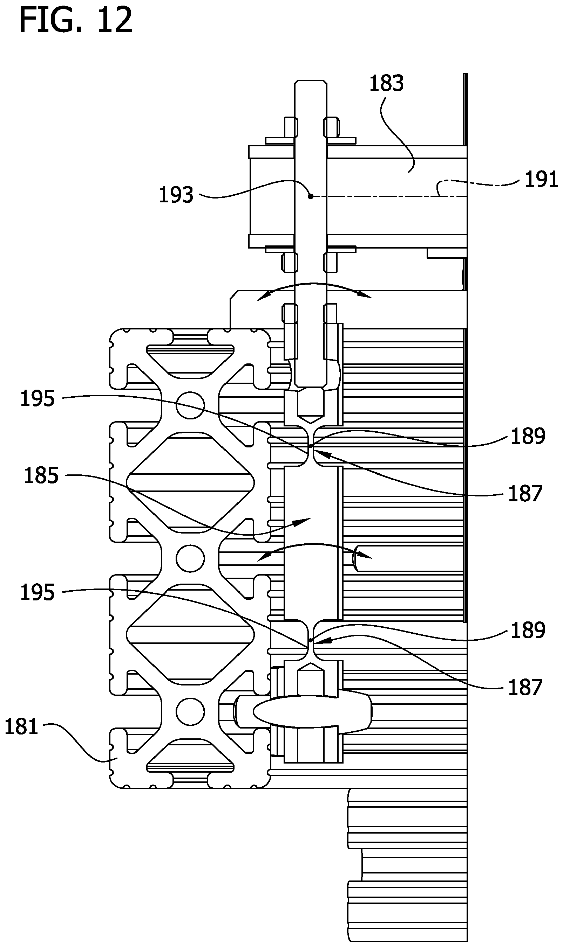

The temperature control block 153 is mechanically isolated from the turntables 131 to facilitate highly precise positioning of the sample containers by the positioning system 121. The frame 181 supports the turntables 131 so they are spaced slightly above the temperature control block 153. Accordingly, there are small gaps 157 between the top of the temperature control block 153 and the bottoms of the turntables 131, as illustrated in Figs. 11 and 12. The gaps 157 are suitably no more than about 0.25 inches in length. In general, heat transfer between the temperature control block 153 and the turntables 131 is better when the gaps 157 are relatively small. The gaps 157 are suitably in the range of about 0.0001 inches to about 0.25 inches and more suitably in the range of about 0.001 inches to about 0.006 inches. In the illustrated example, the gaps 157 are equal in length, but this is not required within the broad scope of the invention. Because of the gaps 157 separating the turntables 131 from the temperature control block 153, there is no physical contact between the turntables and the temperature control block. Consequently, the turntables 131 do not slide on, rub against, or otherwise contact the temperature control block 153 as they move. This helps maintain the frozen samples at the desired temperature because there is no heat from friction between the turntables 131 and the temperature control block 153 when the turntables move.

Although the frame 181 supports the temperature control block 153, the frame is mechanically isolated from the potential effect of thermal expansion and contraction of the temperature control block. This further isolates the turntables 131 (and the positioning system 121 in general) from any thermally-induced strain associated with operation of the temperature control block 153. Referring to Fig. 3, for example, the temperature control block 153 is supported by a plurality of supports 183 (e.g., rectangular bars) connected to the frame 181 by flexure mounts 185. As illustrated in Fig. 12, each flexure mount 185 includes one or more sections 187 adapted to flex generally on an axis 189 generally perpendicular to an imaginary line 191 extending between the connection 193 of the flexure mount to the support 183 and the center 195 of the temperature control block (See Fig. 6). In particular, the flexure mounts 185 are relatively stiff except at the flexible sections 187 and the flexible sections are resistant to bending except along the flexure axis 189. As illustrated in Fig. 12, the flexible sections 187 of flexure mounts include a relatively thin wall 195 or other structure constructed to have a relatively small bending moment about the flexure axis 189 and a relatively higher bending moment in other directions. When the flexure mounts 185 include multiple flexible sections 187, as illustrated, the flexure axes 189 are suitably substantially parallel to one another. Each of the flexure mounts 185 can easily flex at the flexible sections 187 in the directions indicated by the arrows in Fig. 12 as the temperature control block 153 expands or contracts due to thermal changes to alleviate thermal strain while the flexure mounts collectively support the temperature control block 153 securely below the turntables 131.

If desired, the temperature control system 151 can also include one or more chillers or other cooling units (not shown) positioned outside the enclosure 155 surrounding the samples to keep the temperature outside the enclosure relatively cool to minimize the temperature difference between the interior and exterior of the enclosure 155. For example, one or more chillers can be used to cool the air or other gas inside the larger enclosure 105 enclosing the robot 103. In some cases, it may be desirable to keep the temperature outside the inner enclosure 155 and inside the outer enclosure 105 at a significantly higher temperature than the temperature inside the inner enclosure. Thus, the temperature control system can be designed to maintain a temperature outside the inner enclosure 155 but inside the outer enclosure 105 above a particular temperature (e.g., above freezing or at about room temperature). This may be desirable, for instance, to avoid the need to operate any precision motion control devices or other components of the system at the low temperatures maintained inside the enclosure 155.

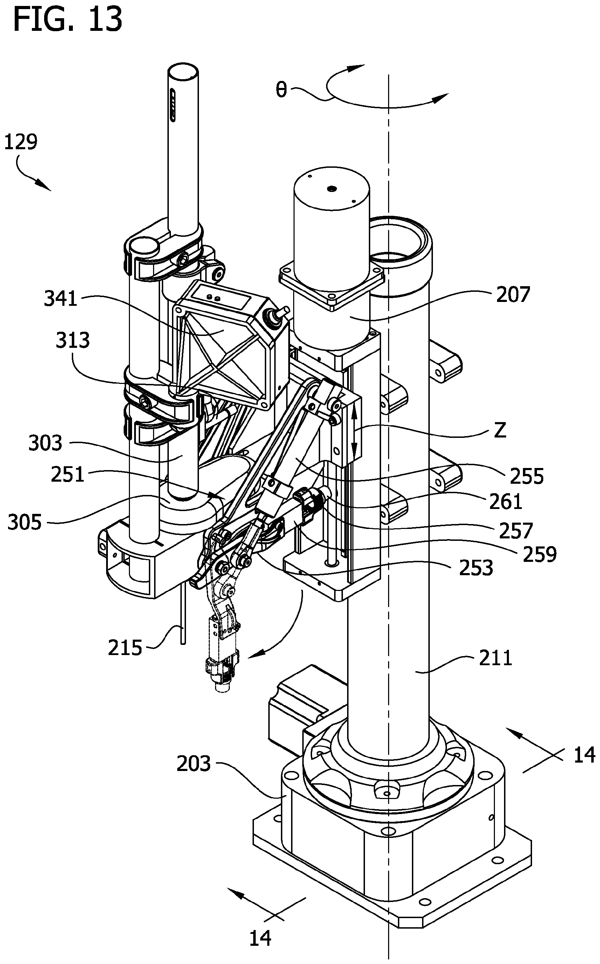

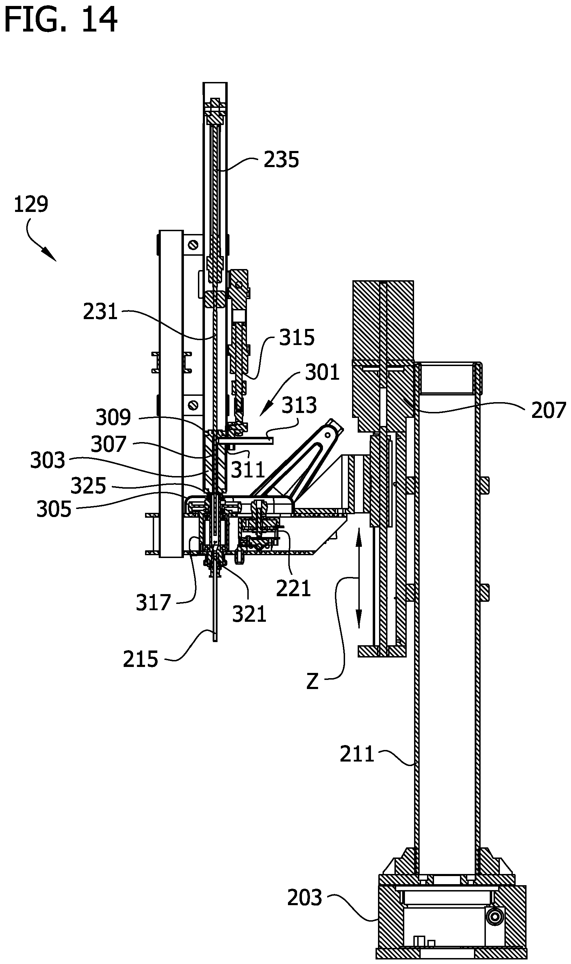

As illustrated in Figs. 13 and 14, the robot arm 129 is mounted on the frame 181 (e.g., at a location outside the inner enclosure 155) for pivoting movement about a vertical axis 201 as indicated by arrows θ. Rotation of the robot arm 129 on this axis 201 is suitably driven by a precision motion control device. As illustrated, for example, a servo-controlled rotary stage 203 mounted on the frame 181 is adapted to drive rotation of the arm 129 on axis 201. Movement of the robot arm 129 in the vertical Z direction is suitably driven by a precision motion control device. For example, movement of the robot arm is suitably driven by a servo-controlled linear stage 207 mounted on a support 211 driven by the rotary stage 203. The rotary stage 203 and linear stage 207 are part of the positioning system 121. Accordingly, in the illustrated example the positioning system 121 has no more than three precision motion control devices (e.g., exactly three precision motion control devices).

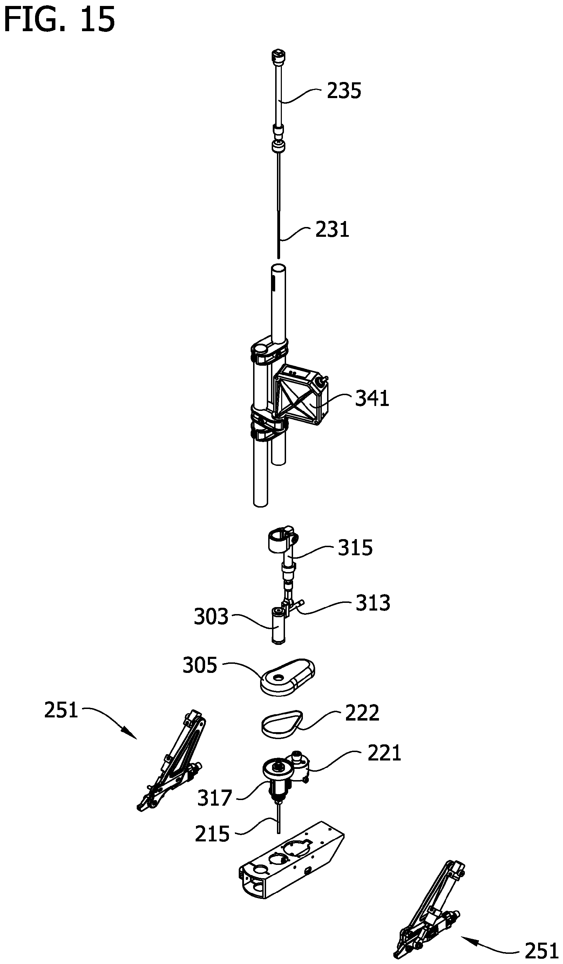

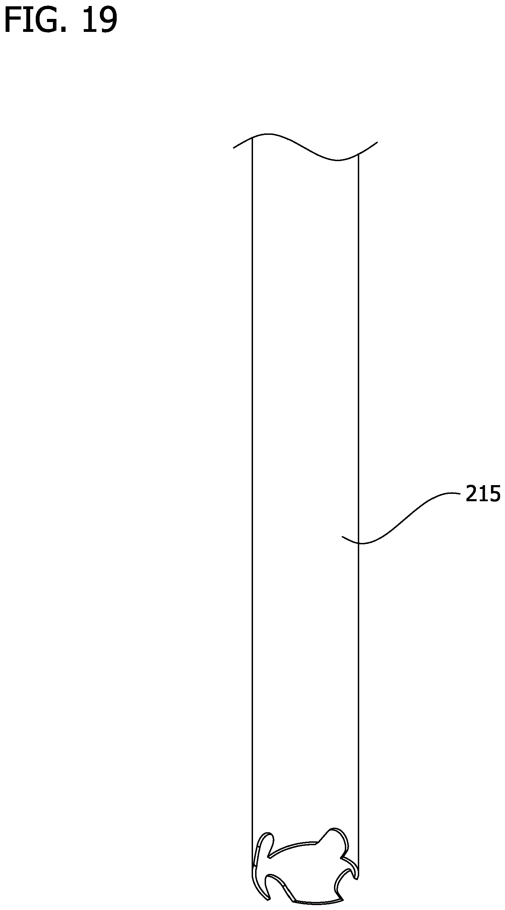

The robot arm 129 suitably includes a sample coring device adapted to take frozen sample cores from the frozen samples by being moved into the frozen samples and then withdrawn from the frozen samples. For example, the robot arm 129 in the illustrated example includes a downward extending hollow coring bit 215 (Fig. 19) and a motor 221 (Figs. 14 and 15) operable to rotate the coring bit. For example, the motor 221 is suitably connected to a rotatable spindle assembly 317 holding the coring bit 215 by a belt (Fig. 15). Although the robot arm 129 in the illustrated example is adapted to rotate the coring bit 215, it is understood there are other ways to operate a drill assembly to obtain a frozen sample core within the broad scope of the invention, including the methods disclosed in U.S. pre-grant publication No. 20090019877.

The motor 221 in the illustrated example is suitably a precision motion control device (e.g., servo-motor) adapted to drive rotation of the coring bit 215. The servo-controlled motor 221, suitably provides very precise control over the speed and torque at which the coring bit is rotated. The ability to control the speed and torque facilitates operation of the coring bit 215 according to various different modes that can be selected to account for the physical characteristics of various types of frozen samples, as will be described in more detail below. Although the coring bit 215 in the example illustrated in the drawings is driven by a precision motion control device 221, this device is not considered part of the positioning system 121 because it only rotates the coring bit and is not involved in moving the containers C or robot arm 129. In the illustrated example, the entire system 101 includes no more than four (e.g., exactly four) precision motion control devices.

The robot arm 129 has a pair of gripping mechanisms 251 on opposite sides of the arm. Each gripping mechanism has a gripping arm 253 pivotally mounted on the robot arm 129 and moveable (e.g., by a pneumatic actuator 253) between a retracted position (shown in solid in Fig. 13) and an extended position (shown in phantom in Fig. 13). The arm 253 has one or more movable fingers 257 at its free end. The fingers 257 are selectively moveable (e.g., by another pneumatic actuator 259) between a hold position in which the fingers are relatively closer to one another and a release position in which the fingers are relatively farther from one another. When the arm 253 is in the extended position, the fingers 257 can be moved to selectively grip or release containers C containing frozen samples or frozen aliquots. For example, the gripping mechanisms 251 can be adapted to grip a cap 261 on the top of a container.

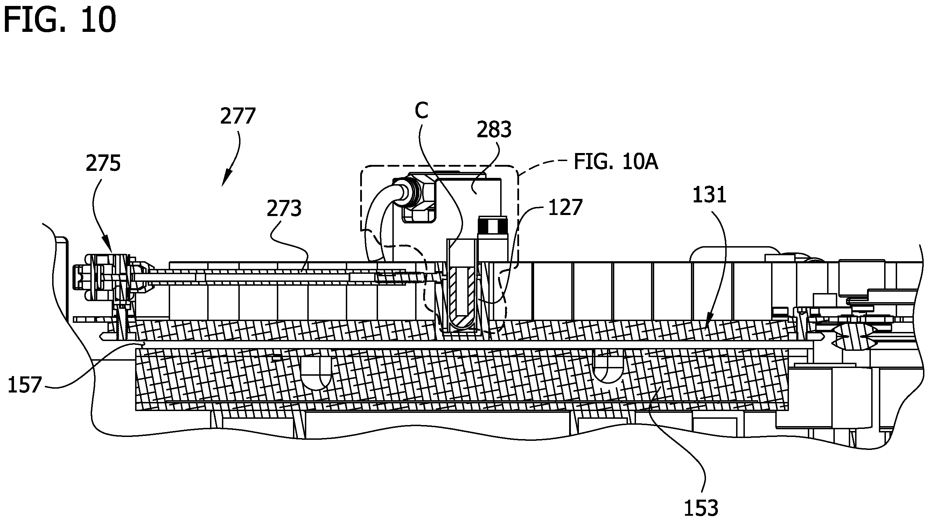

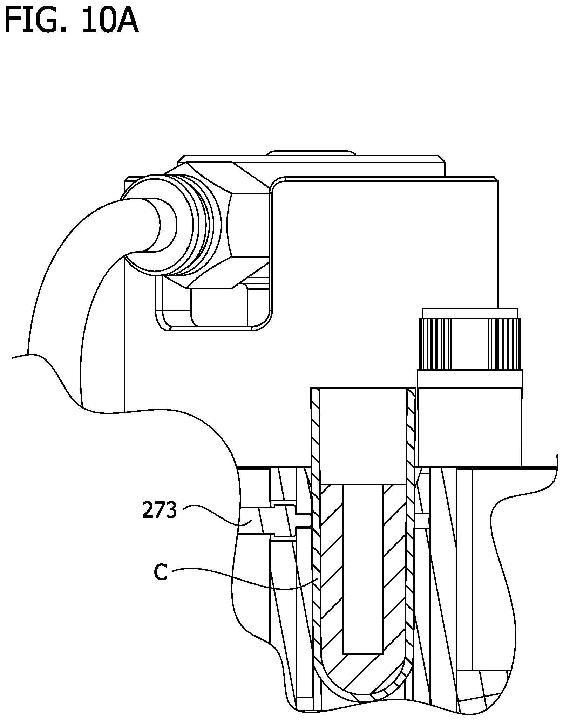

The cap 261 is suitably a threaded cap that can be secured to the container C by screwing the cap onto the top of the container. Thus, when the cap 261 is secured to the container C, the gripping mechanism 251 can pick up the entire container by gripping the cap. The system 101 is also adapted to remove the cap 261 from the container C while it is at the sample coring station 125 or aliquot receiving station 127 without picking up the container. For example, as illustrated in Figs. 10, 10A, 11, 11A, 21, and 22 the system 101 includes a pair of clamping mechanisms 271, each of which is adapted to selectively hold a container C at a respective one of the sample coring and aliquot receiving stations 125, 127 in a fixed position relative to the respective turntable 131 and release the container to permit the container to be moved relative to the turntable.

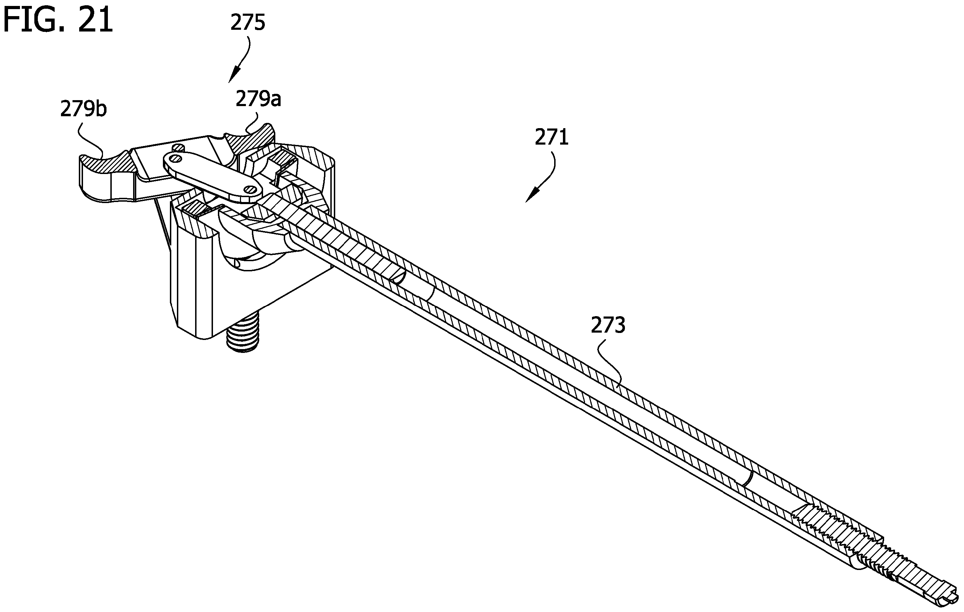

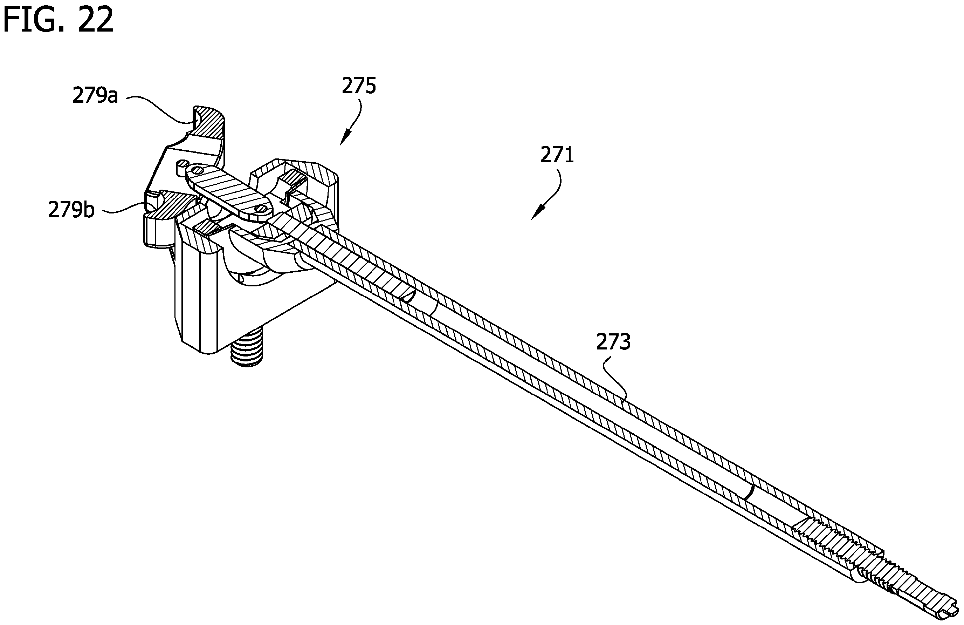

The clamping mechanisms 271 each include a rod 273 extending radially inward toward the sample coring or aliquot receiving station 125, 127 from a toggle mechanism 275 (e.g., mechanical toggle switch) on the perimeter of the respective turntable 131. When the toggle mechanism 275 is in a first position (FIGS. 10, 10A, and 22), the rod 273 extends into the receptacle 133 and clamps the container C in position relative to the turntable 131. When the toggle mechanism 275 is in a second position (Figs. 11, 11A, and 21) the rod 273 does not extend as far toward the center of the turntable 131 as it does in the first position and the rod does not clamp the container C in position. When the container C is not clamped in position by the clamping system 271, the container can easily be rotated relative to the turntable and/or picked up from the turntable 131 (e.g., by the robot arm 129).

The frame 181 supports a pair of clamping mechanism actuators 277 (e.g., pneumatic actuators) positioned on the periphery of the turntables. The actuators 277 can be extended radially inward to move the toggle mechanism 275 between the first and second positions when the turntable is in an orientation such that the portion of the toggle mechanism to be pushed by the actuator to accomplish the desired movement of the toggle mechanism is aligned with the actuator (see alignment of left turntable 131 to actuator 277 in Fig. 8). The toggle mechanisms 275 are each pushed at one location 279a (Figs. 21 and 22) to clamp the container C and another location 279b to release the container. Thus, in the illustrated example, each turntable 131 is at a first orientation (e.g., as illustrated Fig. 8) when the respective actuator 277 is activated to clamp a container and a second orientation different from the first (e.g. rotated slightly counterclockwise from the position illustrated in Fig. 8) when the actuator is activated to release the container.

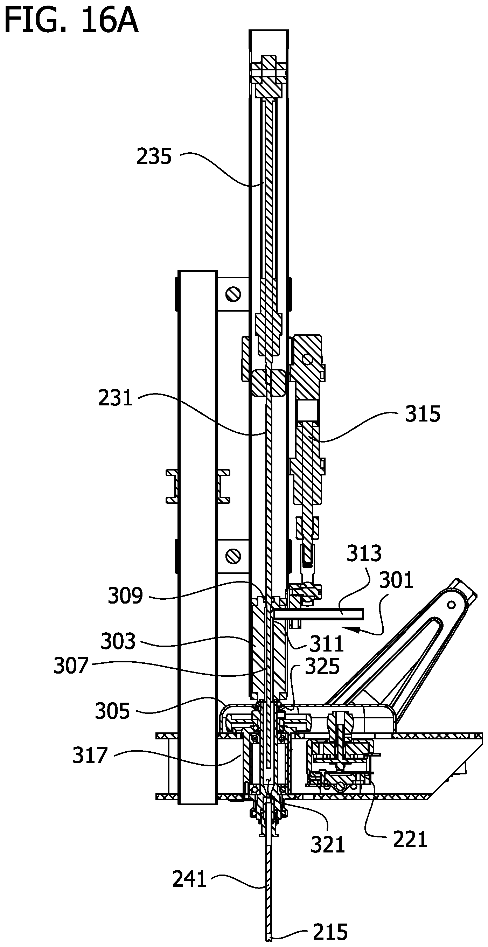

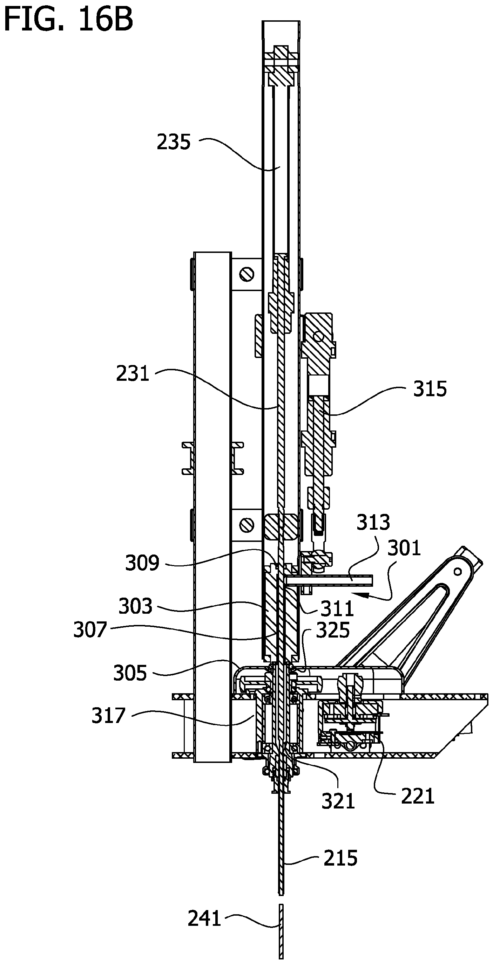

The robot arm 129 also includes a plunger 231 adapted to eject frozen sample cores from the coring bit 215. As illustrated in Figs. 14-16B, the plunger 231 is suitably mounted above the coring bit 215 and connected to a pneumatic actuator 235 adapted to drive movement of the plunger up and down within the robot arm 129. The plunger 231 is normally in a retracted position (Fig. 16A), in which the bottom of the plunger is spaced above the top of the coring bit 215. After a frozen sample core 241 has been taken from one of the frozen samples, the pneumatic actuator 235 drives the plunger 231 downwardly to an extended position (Fig. 16B) to eject the frozen sample core 241 from the hollow coring bit 215 into an aliquot receiving container C.

The enclosure 155 for the frozen samples confines the coldest temperatures in the system 101 to relatively small space surrounding the samples. Significantly, the majority of the robot arm 129 remains outside the enclosure 155 even when the robot arm is inserted into the enclosure to drill into a frozen sample or move sample containers. For example, the motors 203, 207 for moving the robot arm 129 and the motor 221 for driving the coring bit 215 are outside the enclosure 155 in an environment that can be warmer than the environment inside the enclosure without degrading frozen samples. The motor 163 for the turntables 131 is also outside the enclosure. Because the motors 203, 207, 221, and 163 are outside the enclosure 155, they do not need to operate at the lower temperatures existing within the enclosure. For example, the motors 203, 207, 221, and 163 can operate at temperatures above freezing, more suitably at temperatures above about 10 degrees centigrade, and still more suitably at temperatures above about 20 degrees centigrade.

The rollers 197 also are positioned at an outer margin of the enclosure 155. For example, as illustrated, the rollers 197 are spaced radially-outward of the temperature control block 153 where the temperature can be warmer than it is in the immediate vicinity of the temperature control block. As illustrated, the rollers 197 are positioned between the turntable and the enclosure 155. Accordingly, the moving parts of the turntable drive system 161 are positioned at the outer margin of the enclosure 155 or outside the enclosure. Accordingly, the rollers 197 and the rest of the turntable drive system 161 can operate at the warmer temperatures existing outside the enclosure 155 or at the margin of the enclosure instead of at the lower temperatures that may exist in the immediate vicinity of the temperature control block 153 and the frozen samples.

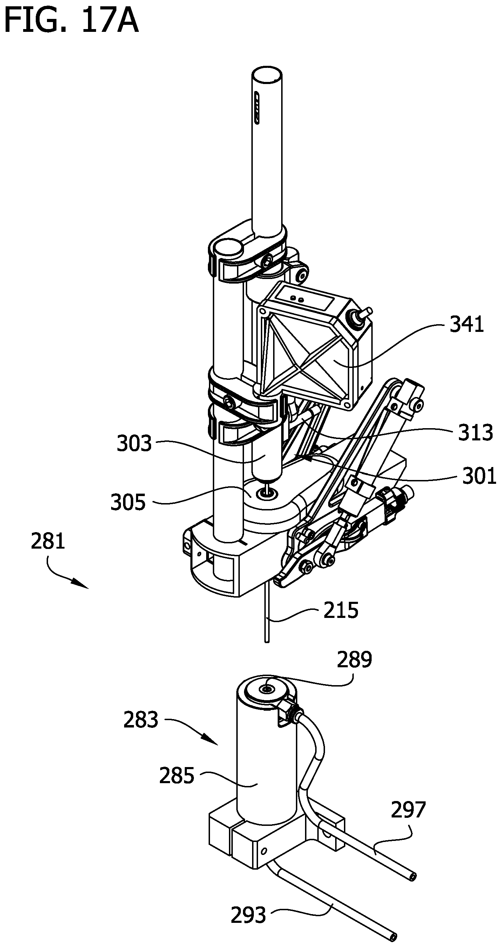

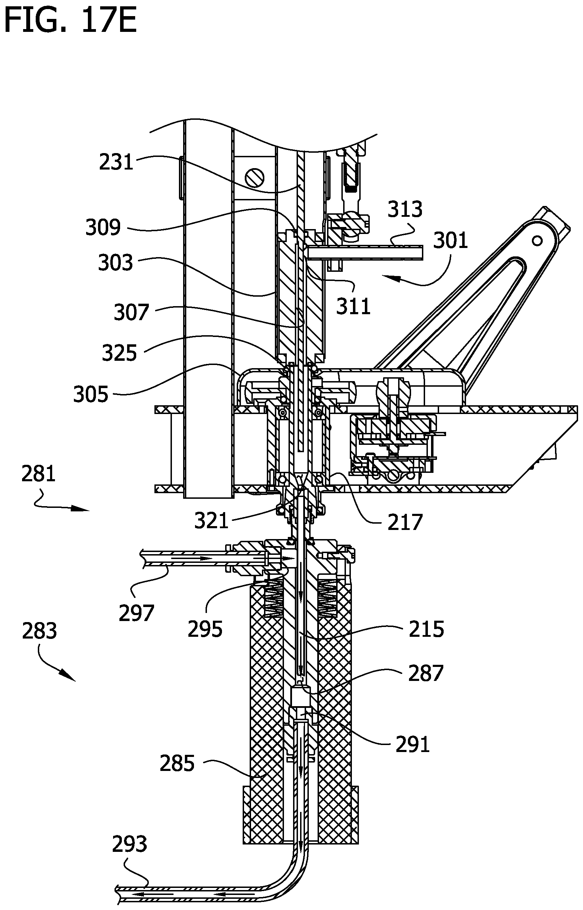

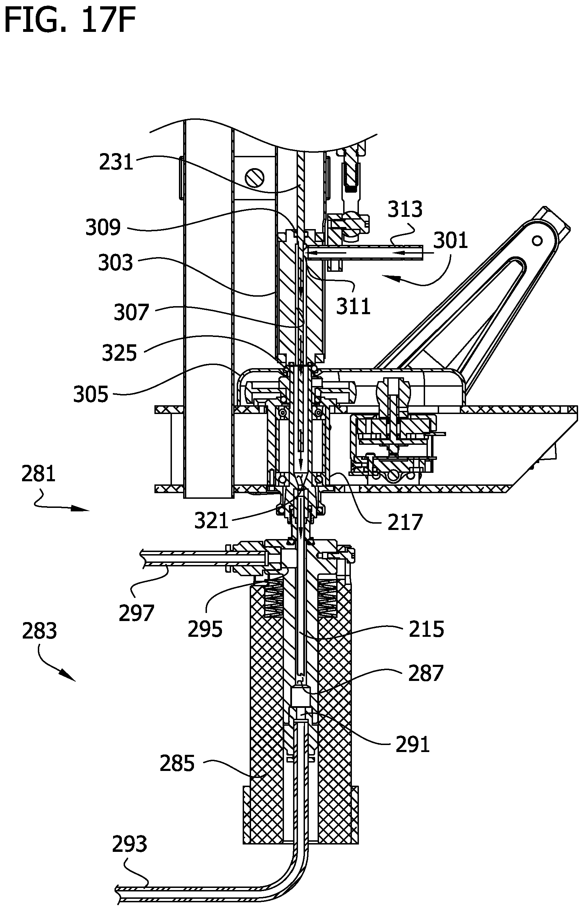

Referring to Figs. 17A-17F, the system 101 includes a cleaning system 281 operable to automatically clean and dry the inside and outside of the coring bit 215 and also clean and dry the lower end of the plunger 231. The cleaning system 281 includes a cleaning station 283 supported by the frame 181. The cleaning station 283 includes a housing 285 having an internal chamber 287 and an opening 289 for inserting the coring bit 215 into the chamber. A drain 291 is installed at the bottom of the chamber 287 and connected to a drain line 293 for draining fluids from the chamber. The housing 285 also has an inlet 295 for receiving one or more cleaning fluids (e.g., a cleaning solution and a rinsing fluid) from a cleaning fluid supply line 297 connected to the housing at the inlet. The cleaning station 238 can optionally include a vibrator (e.g., acoustic or ultrasonic vibrator) (not shown) positioned to agitate cleaning fluid while it is in contact with the coring bit 215 to help dislodge sample materials from the coring bit. For example, the vibrator can be used to help dislodge proteins or other sticky substances from the coring bit 215.

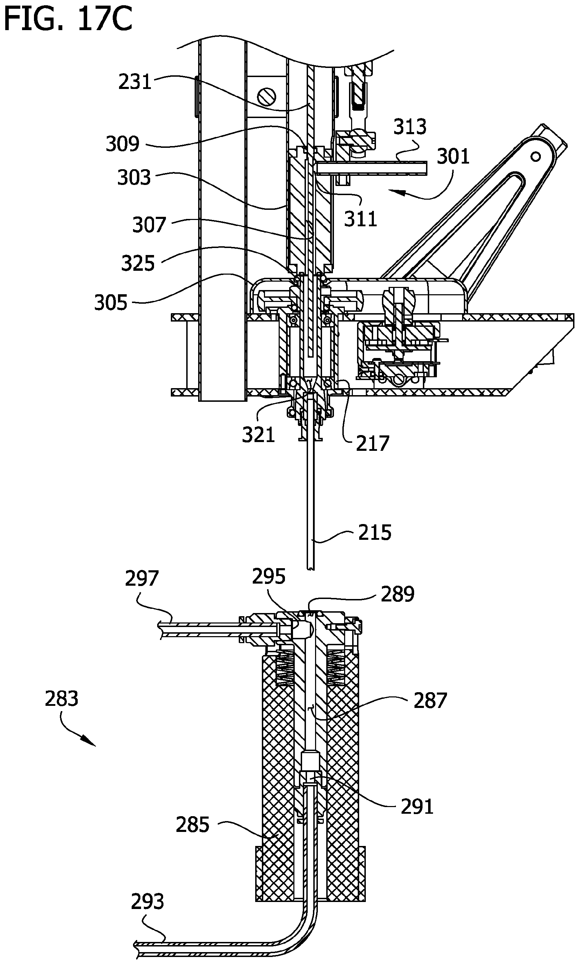

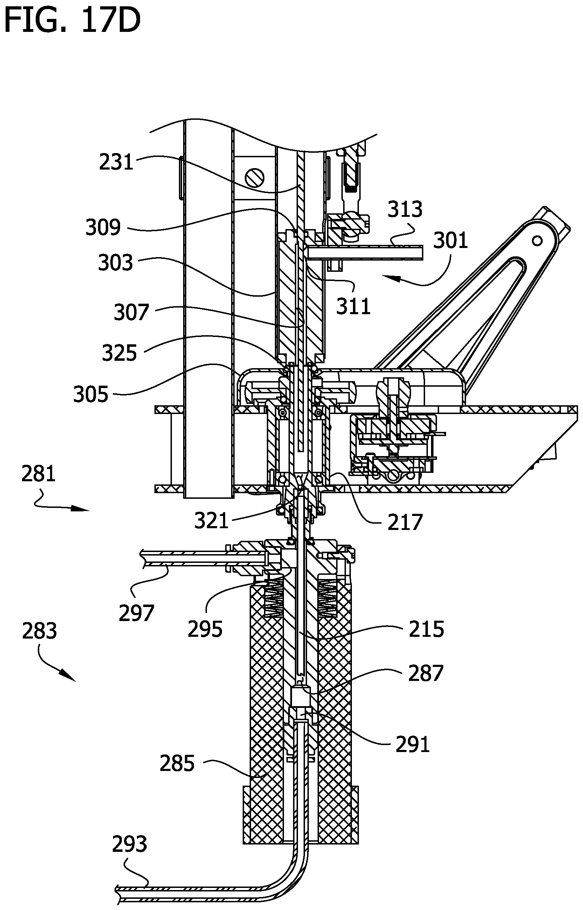

The cleaning system 281 also includes a plunger cleaning sub-system 301 on the robot arm 129. The plunger cleaning sub-system 301 includes a plunger seal tube 303 positioned above the motor housing 305. The plunger 231 extends through a hollow center 307 of the plunger seal tube 303. The plunger seal tube 303 has a fluid inlet 311 for receiving one or more cleaning fluids into its hollow center 307 from a fluid supply line 313 connected to the fluid inlet. A sealing member 309 (e.g., an O-ring) at the top of the plunger seal tube 303 seals against the plunger 231 and limits fluid from flow from the hollow center 307 of the plunger seal tube 303 out the plunger seal tube through its top. The plunger seal tube 303 is selectively moveable by an actuator 315 (e.g., a pneumatic actuator) between a position in which the plunger seal tube is spaced slightly above a rotating spindle assembly 317 (Fig. 17C) that holds the coring bit 215 and a position in which the bottom of the plunger seal tube contacts the spindle assembly (Fig. 17D).

The spindle assembly 317 has a hollow central portion 321 extending from the top of the spindle assembly to the coring bit 215 that is in fluid communication with the hollow center of the coring bit. When the plunger seal tube 303 is moved into contact with the spindle assembly 317, the hollow center 307 of the plunger seal tube is in fluid communication (e.g., aligned with) the hollow central portion 321 of the spindle assembly 317 so fluid in the plunger seal tube can flow down into the spindle assembly and out through the hollow center of the coring bit 215. A sealing member 325 (e.g., an O-ring) forms a seal between the plunger seal tube 303 and the spindle assembly 317 when the plunger seal tube is in contact with the spindle assembly, to limit flow of fluid out of the plunger seal tube except through the spindle assembly. When the plunger seal tube 303 is in the position in which it does not contact the spindle assembly 317, the spindle assembly can rotate with the coring bit 215 without subjecting the sealing member 325 to any sliding or rubbing.

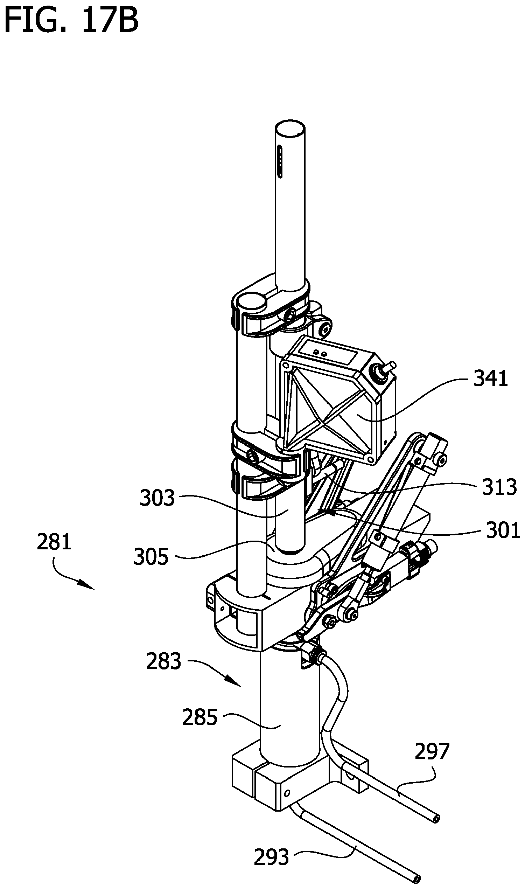

To use the cleaning system 281, the robot arm 129 moves the coring bit 215 into alignment with the opening 289 in the top of the cleaning station 283 (see Fig. 17A and 17C). Then the robot arm 129 moves the coring bit 215 down so the lower end of the bit extends into the chamber 287 (see Figs. 17B and 17D). In this position, the bottom of the spindle assembly 317 forms a seal against the housing 285 of the cleaning station. The plunger seal tube actuator 315 moves the plunger seal tube 303 down into contact with the upper portion of the spindle assembly 317 so the sealing member 325 forms a seal between the plunger seal tube and spindle assembly, as illustrated in Fig. 17D.

To clean the outside of the coring bit 215, cleaning fluid is pumped from a cleaning fluid supply (not shown) through the inlet 295 to inject cleaning fluid into the chamber 287 where it contacts the bit. After contacting the outside of the bit 215, the cleaning fluid exits the chamber through the drain 291. To clean the plunger 231 and inside of the coring bit 215, cleaning fluid is pumped from the same or a different cleaning fluid supply into the robot arm 129 through the plunger seal tube inlet 311 to inject cleaning fluid into the hollow center 307 of the plunger seal tube 303 where it contacts the plunger 231. The cleaning fluid flows from the plunger seal tube 303 into the hollow center 321 of the spindle assembly moving down along the plunger 231, cleaning it as it goes. The cleaning fluid then flows down through the hollow center of the coring bit 215 to clean the inside of the bit. After flowing out of the coring bit 215 into the chamber 287 of the cleaning station, the cleaning fluid is drained through the drain 291. The cleaning fluid is suitably alternately pumped to the cleaning station 283 to clean the outside of the coring bit 215 and then pumped to the robot arm 129 to clean the plunger 231 and inside of the bit 215. However, if desired cleaning fluid can be pumped to the cleaning station 283 and robot arm 129 concurrently within the scope of the invention. At any time during cleaning, the optional vibrator can be activated to agitate the cleaning fluid to help clean the coring bit 215. In some cases, the cleaning fluid may be clean water or another fluid that does not need to be rinsed off the coring bit 215 or plunger 231. If the cleaning fluid is something other than pure water, the cleaning fluid can be rinsed from the coring bit 215 and plunger 2313 if desired.

The cleaning system 281 optionally dries the coring bit 215 after cleaning it with the cleaning fluid. A drying gas (e.g., air, nitrogen, or other suitable gas) is suitably pumped from a drying gas supply (not shown) through the same fluid lines as the cleaning to inject drying gas into the chamber of the cleaning station and into the hollow center of the plunger seal tube to dry the inside and outside of the coring bit. When the sample is to be subjected to quantitative analysis it is important to dry the coring bit 215 after cleaning to prevent dilution of the frozen samples with the cleaning fluid. It is understood, however, the cleaning system is not required to dry the coring bit within the broad scope of the invention.

The system 101 includes a sample inspection system 341 adapted to detect one or more locations within a frozen sample from which a frozen sample core has already been taken. The sample inspection system can include any of a variety of sensors that can be adapted to detect holes in the sample from which frozen cores have already been taken. Suitable sensors and sensor systems that can be used in the sample inspection system 341 include an image analysis system, a vision inspection system, a con-focal imaging system, an optical profilometer, a time-of-flight distance sensor, an angle of incidence/reflection triangulation based distance sensor, and digital camera, and the like. The foregoing sensors and sensor systems can also be used in any combination to increase robustness of the sample inspection system.

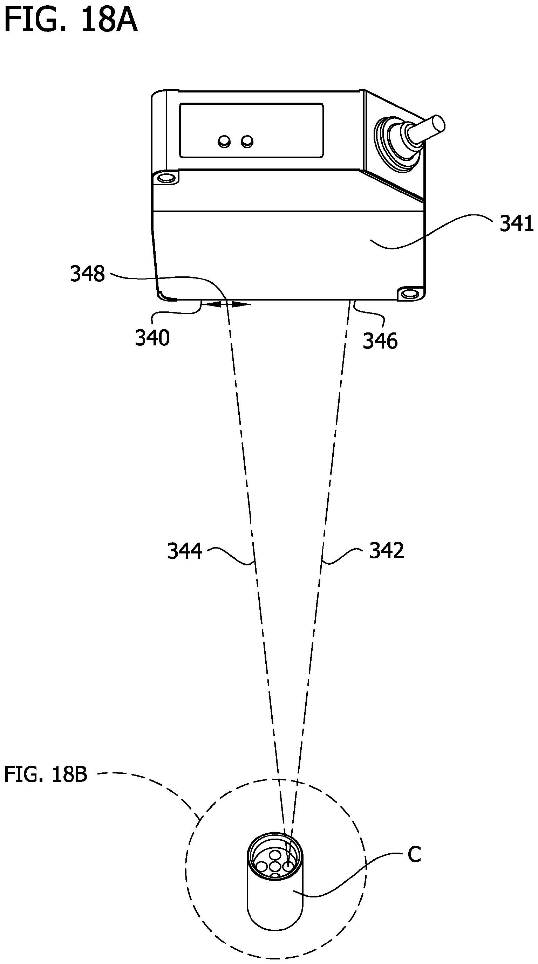

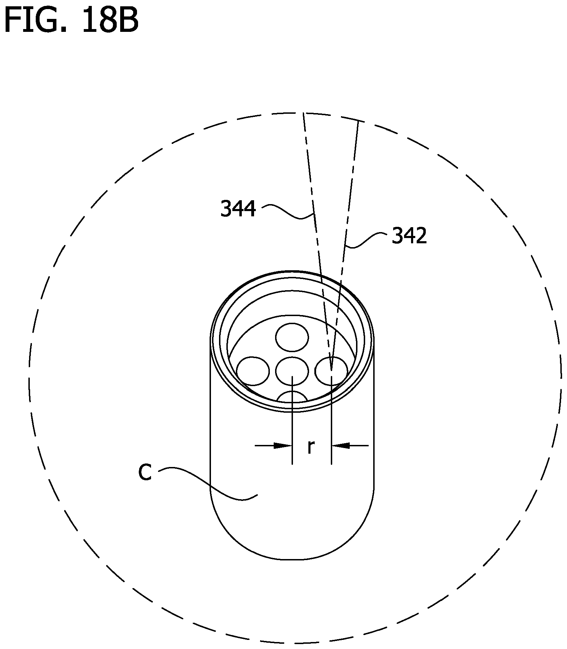

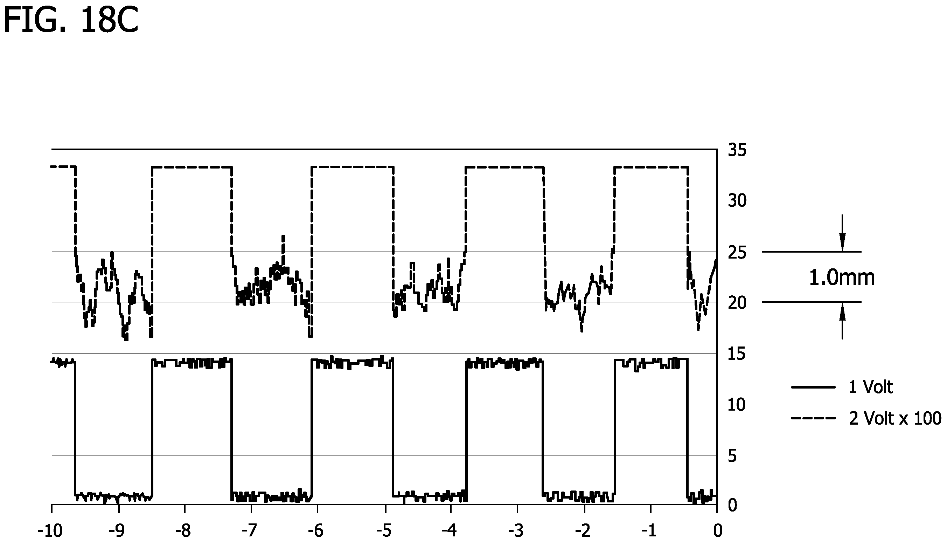

For instance, one example of a sample inspection system suitably includes a precision triangulation-based optical range finder 341 mounted on the robot arm 129. The range finder 341 is operable to direct a beam 342 of electromagnet radiation (e.g., an infrared laser beam) onto the upper surface of a frozen sample at the sample coring station 125 (see Fig. 18A). The range finder 341 also includes a detection system 340 to detect electromagnetic radiation 344 reflected from the surface of the sample and determine the spacing between the location 346 where the beam is emitted and the location 348 on the detector 340 where the reflected beam 344 is received by the detector. By rotating the container C (e.g., using the turntable 131) and/or moving the robot arm 129, the entire upper surface of the frozen sample can be scanned by the beam to identify any locations where frozen sample cores have already been taken. For example, Fig. 18B is a graph illustrating how output from the range finder 341 varies as the beam is scanned over the surface of a sample having four frozen sample core holes arranged in a ring at a radius r from the center of the sample by rotating the container C while the robot arm 129 is positioned so the beam is directed onto the sample at location spaced the same distance r from the center as the sample core holes. When the beam scans over a hole, the spacing between the beam emitter and the location where the reflected beam is received changes (as illustrated by the arrows in Fig. 18A). There is a dramatic jump in the output from the range finder when the beam crosses the edge of a hole, allowing the system to determine the presence of a hole.

Although the triangulation-based optical range finder works well in some cases, the inventors have discovered it does not work well when the physical characteristics of the sample cause it to reflect light in a diffuse manner. Some serum and plasma samples, for example, have characteristics that result in diffuse reflections. When the reflection is diffuse it is difficult or impossible to set the instrument settings so the signal from the sensor can be used to reliably detect any holes in a potentially-cored sample surface. Thus, the sample inspection system desirably includes a sensor that is operable to determine whether or not a potentially-cored surface of the sample has been cored at a particular location regardless of whether or not the sample reflects light in a diffuse manner.

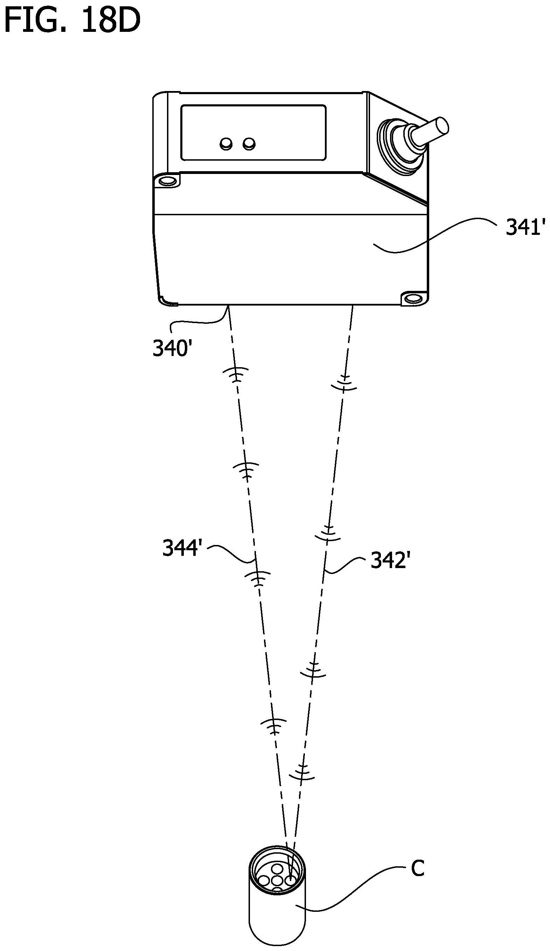

Another example of the sample inspection system includes a time-of-flight based precision range finder 341' (Fig. 18D) The time-of-flight distance sensor comprises a source of electromagnetic radiation adapted to emit a pulsed beam 342' of electromagnetic radiation (e.g., an infrared laser) onto a surface of the sample and a detector 340' adapted to detect radiation reflected by the surface of the sample. The sensor 341' includes or is connected to a processor adapted to output a signal indicative of a difference in time between the time a pulse in the beam 342' is emitted and the time the detector detects the corresponding pulse in the reflected beam 344'. This time will increase slightly as the beam is scanned over a hole in the surface of the sample, allowing output from the sensor to be used to identify the location of any holes in the surface of the sample.

Although the time-of-flight sensor described above uses electromagnetic radiation, it is recognized an analogous acoustic based sensor that emits pulsed acoustic signals and detects echoes from the sample surface could be used in a similar manner within the scope of the invention.

In another example of the system 101, the sample inspection system includes an imaging system (e.g., digital camera not shown) and processor that processes the image to determine if any cores have been taken from a sample. For example, various light/dark contrast algorithms can be used to analyze a complete image of the sample and identify the locations of any holes in the sample surface.

In still another example of the system 101, the sample inspection system includes a processor programmed to analyze a smaller image of only a portion of the sample surface (or only a portion of a larger image of the entire surface) to make a binary decision (yes/no) about whether there are any holes in the sample that would prevent successful coring of the sample at that particular location. If there are no holes in the area immediate surrounding the potential drilling location, the sample inspection system allows the system to drill at that location. On the other hand, if the sample inspection system detects a hole in the vicinity of the potential drilling location, the robot arm and/or container are moved to assess whether or not a different location is suitable for being the location from which a frozen core is taken for use as an aliquot. The simpler yes/no algorithm is much easier to implement and eliminates the need for more elaborate image processing technology required to simultaneously identify the presence and locations of all holes that may exist in a sample.

The system 101 optionally includes a bar code reader 351 (e.g., mounted on the frame 181 as illustrated in Fig. 2) for reading bar code labels that may be applied to the containers C to ensure and verify the aliquots are taken from the correct container and placed in the correct container. It is understood a bar code reader could be positioned elsewhere on the system within the scope of the invention.

To further illustrate the various features of the system 101, one embodiment of a method of using the system to obtain one or more frozen aliquots from frozen samples by taking frozen sample cores will now be described. It is understood that the system 101 can be used in different ways.

To start the process, a plurality of capped containers C containing frozen samples from which one or more aliquots are desired are placed in trays 135 and loaded on one of the turntables 131 (e.g., adjacent the sample coring station 125). A plurality of empty capped containers C for receiving the aliquots are placed in other trays 135 and loaded on one of the turntables 131 (e.g., adjacent the aliquot receiving station 127). For example, all of the frozen sample containers C can suitably be placed on one turntable 131 while all of the aliquot receiving containers are placed on the other turntable. However, it is understood that this is not required.

After the containers C are loaded on the system 101 the robot 103 begins taking the aliquots. The robot arm 129 moves into a position so one of the gripping mechanisms 251 is above a container C containing a frozen sample. The actuator 255 is activated to move the gripping arm 253 to the extended position. Then the robot arm is lowered until the fingers 257 of the gripping mechanism 251 are adjacent the sides of the cap 261. The finger actuator 259 is activated to move the fingers 257 into contact with opposing sides of the cap to grip the cap. The robot arm 129 is raised to lift the container C, moved to a position so the container is above the sample coring station 125, and then lowered to place the container on the sample coring station.

Once the container C is received in the receptacle 133 at the sample coring station 125, the turntables 131 are rotated to align the toggle mechanism actuator 277 with the clamp button 279a on the toggle mechanism 275. The toggle mechanism actuator 277 is extended to push the clamp button 279a on the toggle mechanism and thereby clamp the container at the sample coring station to hold it in a fixed position relative to the turntable 131. While the container C is clamped and the gripping mechanism 251 is still holding the cap, the turntables 131 are rotated to unscrew the cap 261 from the container. The robot arm 129 can be raised as the cap is being unscrewed so the gripping mechanism 251 does not press down on the cap 261 while it is being unscrewed. After the cap 261 is unscrewed, the robot arm 129 is raised further and the gripping arm 253 is retracted by the actuator 255 while still holding the cap.

The process is repeated using the other gripping mechanism to move the aliquot receiving container to the aliquot receiving station 127 on the other turntable 131. At this point, the sample container C is uncapped and clamped in position at the sample coring station 125 and the aliquot receiving container is uncapped and clamped at the aliquot receiving station 127. The caps 261 for the containers are retained by the retracted gripping mechanism.

The sample inspection device 241 is used to inspect the upper surface of the frozen sample in the container C at the sample coring station 125 to determine whether any previous frozen sample cores have been taken from the frozen sample. If frozen sample cores have already been taken from the frozen sample, the sample inspection device 341 also determines the locations within the sample from which the frozen cores have been taken, and by a process of elimination identifies one or more positions from which another frozen core can be taken.

The robot arm 129 moves the coring bit into position above the sample container at the sample coring station 125. In particular, the robot arm 129 moves the bit into a position above a portion of the sample from which no frozen samples cores have been taken yet. In one example, the location from which the frozen sample core is to be taken is selected to be at a radial position where the concentration of at least one substance of interest in the frozen sample core is representative of the overall concentration of said at least one substance of interest in the sample notwithstanding any concentration gradients that may exist in the frozen sample.

The present inventors have recognized that radial concentration gradients can develop in a biological sample as it is being frozen. In order to ensure the concentration of the substance of interest in the aliquot is representative of the concentration of that substance in the original unfrozen sample, it is necessary to take the frozen sample core from a position that is radially offset from the center axis of the sample. The position at which the local concentration in the frozen sample is representative of the overall concentration varies depending on the characteristics of the sample and the substance of interest and is determined experimentally for any particular type of sample and substance of interest.

It is also recognized that two or more frozen sample cores can be taken from different radial positions in the sample that are selected so an aggregate aliquot formed by combining the multiple frozen sample cores has a concentration of a substance of interest that is representative of the concentration of that substance in the original unfrozen sample. For example, a first frozen sample core can be taken from position at which the local concentration of the substance of interest is known to be too high to be representative of the overall sample and a second frozen sample core can be taken from a position at which the local concentration of the substance of interest is too low to be representative of the overall sample. However, the positions from which the first and second sample cores are taken can be selected so when the first and second sample cores are combined in a single aggregate aliquot the concentration of the substance of interest in the aggregate aliquot is representative of the overall concentration of the substance of interest in the original sample.

The high-precision positioning system 121 facilitates taking the frozen sample core from the desired location with a high degree of accuracy. This capability can be important when the aliquots are to be used in quantitative tests (i.e., tests in which the concentration or relative concentration of one or more substances is needed). Those skilled in the art will recognize that for some tests, it is only necessary to know whether or not a particular substance is present and the precise concentration at which it is present is not that important. Accordingly, it is not necessary to take the frozen sample core from any particular position within the sample.

The coring bit motor 221 turns the coring bit 215 as the robot arm 129 is lowered to move the tip of coring bit 215 from the upper surface of the sample down into the frozen sample. In one embodiment of the method, the coring bit 215 is lowered substantially all the way to the bottom of the container C to obtain a frozen sample core extending substantially all the way through the vertical height of the sample at the location from which the frozen sample core is taken. This can be desirable to account for any vertical concentration gradients that may exist in the sample.

Once the drilling process is complete, the motor 221 is deactivated to stop rotation of the coring bit 215. Then the robot arm 129 is raised to lift the coring bit and the frozen sample core contained therein out of the sample container. The robot arm 129 is then moved to position the coring bit 125 and the frozen sample core contained therein to a position above the aliquot receiving container at the aliquot receiving station 127. The plunger actuator 235 is activated to eject the sample core from the coring bit 215 into the aliquot receiving container.

Once the frozen sample core is in the aliquot receiving container, the robot 103 screws the caps 261 back onto the sample container and the aliquot receiving container using the gripping mechanisms 251 to hold the caps stationary while the turntables 131 are rotated to rotate the clamped containers. The robot arm 129 may move down during the process of screwing the cap 261 back on the containers to track downward movement of the cap as it is screwed on the container. After the caps 261 are back on the containers, the turntables 131 are rotated to align the toggle mechanism actuator 277 with the release buttons 279b on the toggle mechanisms 275. The actuators 277 are extended to press the release buttons 279b and unclamp the containers C so they can be removed from the sample coring and aliquot receiving stations 125, 127. Then the robot arm 129 is moved to position the gripping mechanisms 251 over the containers C to pick them up and move them back to the temporary storage positions 123 on the turntables. In this embodiment of the method, the containers C are only uncapped at the sample coring station 125 or aliquot receiving station 127. The containers C are always capped when they are at any of the storage positions 123.

If more than one aliquot is needed from the same sample, the process is substantially the same except the robot 103 leaves the sample container C at the sample coring station 125 while it removes the first aliquot receiving container from the aliquot receiving station 127 and replaces it with another aliquot receiving container. Then the robot 103 obtains another frozen sample core from the sample and places it in the second aliquot receiving container. This process can be repeated to obtain three, four or more aliquots from a single sample during one aliquotting session, as long as there is sufficient sample material for the aliquots. It is also possible to place multiple frozen sample cores into a single aliquot receiving container (e.g., to obtain a larger volume of sample material).

The cleaning system 281 is then used to clean the coring bit 215 and plunger 231 in the manner described above. After cleaning, the process can be repeated with a different sample. After all the aliquots to be taken from the samples loaded on the system 101 have been taken, the trays 135 are removed from the turntables. The still frozen samples are returned to the cryogenic storage so they will be available in the future if there is a desire to obtain another aliquot for use in a different test. The aliquots are taken from the system 101 and delivered to customers of the biobank or biorepository for testing.

It will be appreciated from the foregoing that the system 101 is able to accomplish one or more (e.g., all) of the following tasks using only three precision motion control devices (the servo-controlled turntable motor 161, the servo-controlled rotary stage 203 for moving the robot arm 129 in the θ direction, and the servo-controlled linear stage 207 for moving the robot arm in the z-direction) to control positioning of the relevant structures:
(a) move a container from a temporary storage location 123 to a sample coring station 125;
(b) move another container from the temporary storage location to an aliquot receiving station 127 spaced from the sample coring station;
(c) activate and release clamping mechanisms 271 to hold and release the containers in fixed positions at the sample coring and aliquot receiving stations;
(d) remove threaded caps from the containers;
(e) scan the beam from the sample inspection device 341 across the upper surface of the frozen sample to locate any positions in the sample from which sample cores have already been taken;
(f) move a sample coring device (e.g., coring bit 215) into the sample container to obtain a frozen sample core from a location that has not been previously cored;
(g) transfer the frozen sample core to the aliquot receiving container at the aliquot receiving station;
(h) screw the threaded caps back onto the containers;
(i) move the containers back from the sample coring and aliquot receiving stations to the temporary storage location; and
(j) move the sample coring device to a cleaning station.

Another feature of the system 101 is the processor is suitably adapted to accept input from a user and operate the system in one of multiple different modes selected by the user For example, the processor is suitably programmed and/or hardwired to operate in various modes differing from one another in one of more of the following parameters:
(a) a speed at which the robot 103 moves the coring bit 215 axially into the frozen samples to obtain the sample cores;
(b) a force with which the robot 103 moves the coring bit 215 axially into the frozen samples to obtain the sample cores;
(c) a speed at which the robot 103 rotates the coring bit 215 to obtain the sample cores;
(d) a torque applied to the coring bit to obtain the sample cores;
(e) an amount of an impact force applied to the coring bit 215 as it is moved axially into the frozen samples to obtain the sample cores;
(f) a position within each of the respective samples from which the frozen sample cores are taken;
(g) a depth to which the sample coring device is moved into the frozen sample; and
(h) a size or shape of a drill bit used by the sample coring device to take the frozen sample core.

The ability to operate in different modes allows users the flexibility to select a mode designed to facilitate taking frozen sample cores from the frozen samples while limiting the risk and/or extent of physical damage to the sample resulting from the coring process. The variables listed above for the modes can influence the propensity of the frozen sample to crack or otherwise break, be heated by friction, partially melt, or experience other effects that degrade the sample and/or limit the number of frozen sample cores that can be taken from a particular frozen sample. The optimal settings for the variables identified above can vary depending on the characteristics of the frozen sample, the characteristics of the container, the temperature of the frozen samples, and other variables. For example, one mode of operation can be adapted to facilitate taking aliquots from frozen serum sample cores without cracking or otherwise damaging the frozen serum samples and another mode can be adapted to facilitate taking frozen sample cores from frozen plasma samples without cracking or otherwise damaging the frozen plasma samples.

Having described the invention in detail, it will be apparent that modifications and variations are possible without departing from the scope of the invention defined in the appended claims.

When introducing elements of the present invention or the preferred embodiment(s) thereof, the articles "a", "an", "the", and "said" are intended to mean that there are one or more of the elements. The terms "comprising", "including", and "having" are intended to be inclusive and mean that there may be additional elements other than the listed elements.

## Claims

1. A method of obtaining an aliquot (241) of a frozen sample contained in a cylindrical container (C), the sample being a mixture comprising two or more substances, the concentration of at least one substance of interest having a radial concentration gradient, the method comprising;
moving a sample coring device (215) into the frozen sample at a location and then withdrawing the sample coring device (215) from the sample to obtain the aliquot (241) in the form of a frozen sample core (241) taken from said location,
**characterized in that** the method further comprises:
experimentally determining for the same type of sample and a substance of interest, the radial position (r) at which the concentration of said substance of interest in the frozen sample is representative of the overall concentration of said substance of interest, and
selecting said location to be at a radial position (r) where the concentration of said substance of interest in the frozen sample core (241) is representative of the overall concentration of said substance of interest in the sample.

2. A method as set forth in claim 1 wherein said location (r) is offset radially from a geometric center of the sample.

3. A method as set forth in any one of claims 1 and 2 wherein moving the sample coring device (215) into the frozen sample comprises moving the sample coring device (215) substantially all the way from one side of the sample to an opposite side of the sample at said location (r).

4. A method as set forth in any one of claims 1-3 wherein the sample comprises a substance selected from the group consisting of plasma, serum, urine, whole blood, cord blood, other blood-based derivatives, cerebral spinal fluid, mucus, ascites, saliva, amniotic fluid, seminal fluid, tears, sweat, sap or other plant-derived fluid, animal cells, plant cells, protozoal cells, fungal cells, bacterial cells, buffy coat cells, cell lysates, cell homogenates, cell suspensions, microsomes, cellular organelles, nucleic acids, small molecule compounds in suspension or solution.

5. A method as set forth in claim 4 wherein the sample comprises a substance selected from the group consisting of serum and plasma.

6. A method as set forth in any one of claims 1-5 further comprising using a sample inspection system (341) to identify whether or not any frozen sample cores (214) have already been taken from the sample, said location (r) being selected to be at a position where no frozen sample cores (214) have previously been taken from the sample.

7. A robotic system (101) programmed or hardwired to implement the method set forth in any one of claims 1-6.

8. A method of obtaining an aliquot (214) of a frozen sample contained in a cylindrical container (C), the sample being a mixture comprising two or more substances, the concentration of at least one substance of interest having a radial concentration gradient, the method comprising:
moving a sample coring device (215) into the frozen sample at a first location and then withdrawing the sample coring device from the sample to obtain a first aliquot (214) in the form of a frozen sample core (214) taken from said first location, and
moving the sample coring device (215) or another sample coring device (215) into the frozen sample at a second location having a different radial position within the sample from the first location and then withdrawing the sample coring device (215) from the sample to obtain a second aliquot (214) in the form of a frozen sample core (214) taken from said second location,
combining the first and second aliquots (214) to form an aggregate aliquot, **characterized in that** the method further comprises:
experimentally determining for the same type of sample and a substance of interest, the radial position at which the concentration of said substance of interest in the aggregate aliquot is representative of the overall concentration of said substance of interest, and
selecting the first and second locations so the concentration of said substance of interest in the aggregate aliquot is representative of the overall concentration of said substance of interest in the sample.

## Patentansprüche

1. Verfahren zum Erhalten einer Aliquote (241) einer gefrorenen in einem zylindrischen Behälter (C) enthaltenen Probe, wobei die Probe eine Mischung ist, die zwei oder mehr Stoffe umfasst, wobei die Konzentration wenigstens eines Stoffes von Interesse einen radialen Konzentrationsgradienten aufweist, wobei das Verfahren Folgendes umfasst;
Bewegen einer Probenkernentnahmevorrichtung (215) an einer Stelle in die gefrorene Probe hinein und dann Zurückziehen der Probenkernentnahmevorrichtung (215) aus der Probe, um die Aliquote (241) in der Form eines gefrorenen Probenkerns (241) zu erhalten, der von der Stelle entnommen wird, **dadurch gekennzeichnet, dass** das Verfahren ferner Folgendes umfasst:
experimentelles Bestimmen der radialen Position (r), an der die Konzentration des Stoffes von Interesse in der gefrorenen Probe für die Gesamtkonzentration des Stoffes von Interesse darstellend ist, für denselben Probentyp und für den Stoff von Interesse, und
Auswählen der Stelle, damit sie an einer radialen Position (r) ist, an der die Konzentration des Stoffes von Interesse in dem gefrorenen Probenkern (241) für die Gesamtkonzentration des Stoffes von Interesse in der Probe darstellend ist.

2. Verfahren nach Anspruch 1, wobei die Stelle (r) von einer geometrischen Mitte der Probe radial versetzt ist.

3. Verfahren nach einem der Ansprüche 1 und 2, wobei das Bewegen der Probenkernentnahmevorrichtung (215) in die gefrorene Probe hinein das Bewegen der Probenkernentnahmevorrichtung (215) im Wesentlichen vollständig von einer Seite der Probe zu einer gegenüberliegenden Seite der Probe an derselben Stelle (r) umfasst.

4. Verfahren nach einem der Ansprüche 1-3, wobei die Probe einen Stoff umfasst, der aus der Gruppe ausgewählt ist, bestehend aus Plasma, Serum, Urin, Vollblut, Nabelschnurblut, anderen blutbasierten Derivaten, Rückenmarksflüssigkeit, Schleim, Aszitesflüssigkeit, Speichel, Fruchtwasser, Samenflüssigkeit, Tränen, Schweiß, Saft oder einer anderen aus Pflanzen gewonnenen Flüssigkeit, tierischen Zellen, Pflanzenzellen, Protozoen, Pilzzellen, Bakterienzellen, Leukozyten, Zelllysaten, Zellhomogenaten, Zellsuspensionen, Mikrosomen, Zellorganellen, Nukleinsäuren, niedermolekularen Verbindungen in einer Suspension oder einer Lösung.

5. Verfahren nach Anspruch 4, wobei die Probe einen Stoff umfasst, der aus der Gruppe bestehend aus Serum und Plasma ausgewählt ist.

6. Verfahren nach einem der Ansprüche 1-5, ferner umfassend das Verwenden eines Probenuntersuchungssystems (341), um zu identifizieren, ob einer der gefrorenen Probenkerne (214) bereits aus der Probe entnommen wurde oder nicht, wobei die Stelle (r) derart ausgewählt wird, dass sie sich an einer Position befindet, an der zuvor keine gefrorenen Probenkerne (214) aus der Probe entnommen wurden.

7. Robotersystem (101), das programmiert oder festverdrahtet ist, um das Verfahren nach einem der Ansprüche 1-6 umzusetzen.

8. Verfahren zum Erhalten einer Aliquote (214) einer gefrorenen in einem zylindrischen Behälter (C) enthaltenen Probe, wobei die Probe eine Mischung ist, die zwei oder mehr Stoffe umfasst, wobei die Konzentration wenigstens eines Stoffes von Interesse einen radialen Konzentrationsgradienten aufweist, wobei das Verfahren Folgendes umfasst:
Bewegen einer Probenkernentnahmevorrichtung (215) an einer ersten Stelle in die gefrorene Probe hinein und dann Zurückziehen der Probenkernentnahmevorrichtung aus der Probe, um eine erste Aliquote (214) in der Form eines gefrorenen Probenkerns (214) zu erhalten, der von der ersten Stelle entnommen wird, und
Bewegen der Probenkernvorrichtung (215) oder einer anderen Probenkernvorrichtung (215) an einer zweiten Stelle, die eine sich von der ersten Stelle unterscheidende radiale Position innerhalb der Probe aufweist, in die gefrorene Probe hinein und dann Zurückziehen der Probenkernentnahmevorrichtung (215) aus der Probe, um eine zweite Aliquote (214) in der Form eines gefrorenen Probenkerns (214) zu erhalten, der von der zweiten Stelle entnommen wird,
Kombinieren der ersten und der zweiten Aliquote (214), um eine aggregierte Aliquote auszubilden, **dadurch gekennzeichnet, dass** das Verfahren ferner Folgendes umfasst:
experimentelles Bestimmen der radialen Position, an der die Konzentration des Stoffes von Interesse in der aggregierten Aliquote für die Gesamtkonzentration der den Stoff von Interesse darstellend ist, für denselben Probentyp und für den Stoff von Interesse, und Auswählen der ersten und der zweiten Stelle derart, dass die Konzentration des Stoffes von Interesse in der aggregierten Aliquote für die Gesamtkonzentration des Stoffes von Interesse in der Probe darstellend ist.

## Revendications

1. Procédé d'obtention d'une aliquote (241) d'un échantillon congelé contenu dans un récipient cylindrique (C), l'échantillon étant un mélange contenant au moins deux substances, la concentration d'au moins une substance d'intérêt présentant un gradient de concentration radial, le procédé consistant à :
déplacer un dispositif de carottage d'échantillons (215) dans l'échantillon congelé à un emplacement donné puis à retirer le dispositif de carottage d'échantillons (215) de l'échantillon pour obtenir l'aliquote (241) sous forme d'un noyau d'échantillon congelé (241) extrait dudit endroit, **caractérisé en ce que** le procédé consiste en outre à :
déterminer expérimentalement, pour le même type d'échantillon et une substance d'intérêt, la position radiale (r) à laquelle la concentration de ladite substance d'intérêt dans l'échantillon congelé est représentative de la concentration globale de ladite substance d'intérêt, et
sélectionner ledit emplacement pour qu'il soit à une position radiale (r) où la concentration de ladite substance d'intérêt dans le noyau d'échantillon congelé (241) est représentative de la concentration globale de ladite substance d'intérêt dans l'échantillon.

2. Procédé selon la revendication 1 dans lequel ledit emplacement (r) est radialement décalé d'un centre géométrique de l'échantillon.

3. Procédé selon l'une quelconque des revendications 1 et 2 dans lequel déplacer le dispositif de carottage d'échantillons (215) dans l'échantillon congelé consiste à déplacer le dispositif de carottage d'échantillons (215) substantiellement tout du long d'un côté de l'échantillon à un côté opposé de l'échantillon audit emplacement (r).

4. Procédé selon l'une quelconque des revendications 1 à 3 dans lequel l'échantillon contient une substance choisie dans le groupe constitué de plasma, sérum, urine, sang entier, sang ombilical, autres dérivés du sang, fluide cérébrospinal, mucus, ascites, salive, liquide amniotique, fluide séminal, larmes, sueur, suc ou autres fluides végétaux, cellules animales, cellules végétales, cellules protozoaires, cellules fongiques, cellules bactériennes, cellules de couche leucoplaquettaire, lysats cellulaires, homogénats cellulaires, suspensions cellulaires, microsomes, organelles cellulaires, acides nucléiques, composés à petites molécules en suspension ou en solution.

5. Procédé selon la revendication 4 dans lequel l'échantillon comprend une substance choisie dans le groupe constitué de sérum et de plasma.

6. Procédé selon l'une quelconque des revendications 1 à 5 comprenant en outre d'utiliser un système d'inspection d'échantillon (341) pour identifier si des noyaux d'échantillon congelés (214) ont ou non déjà été extraits de l'échantillon, ledit emplacement (r) étant choisi pour être à une position où aucun noyau d'échantillon congelé (214) n'a été précédemment extrait de l'échantillon.

7. Système robotique (101) programmé ou câblé pour mettre en oeuvre le procédé selon l'une quelconque des revendications 1 à 6.

8. Procédé d'obtention d'une aliquote (214) d'un échantillon congelé contenu dans un récipient cylindrique (C), l'échantillon étant un mélange contenant au moins deux substances, la concentration d'au moins une substance d'intérêt ayant un gradient de concentration radial, le procédé consistant à :
déplacer un dispositif de carottage d'échantillons (215) dans l'échantillon congelé à un premier emplacement donné puis à retirer le dispositif de carottage d'échantillons de l'échantillon pour obtenir une première aliquote (214) sous forme d'un noyau d'échantillon congelé (214) extrait dudit premier emplacement, et
déplacer un dispositif de carottage d'échantillons (215) ou un autre dispositif de carottage d'échantillons (215) dans l'échantillon congelé à un second emplacement ayant une position radiale différente du premier emplacement dans l'échantillon puis retirer le dispositif de carottage d'échantillons (215) de l'échantillon pour obtenir une second aliquote (214) sous forme d'un noyau d'échantillon congelé (214) extrait dudit second emplacement,
combiner les première et seconde aliquotes (214) pour former une aliquote agrégée, **caractérisé en ce que** le procédé consiste en outre à :
déterminer expérimentalement, pour le même type d'échantillon et une substance d'intérêt, la position radiale à laquelle la concentration de ladite substance d'intérêt dans l'aliquote agrégée est représentative de la concentration globale de ladite substance d'intérêt, et à choisir les premier et second emplacements de façon que la concentration de ladite substance d'intérêt dans l'aliquote agrégée soit représentative de la concentration globale de ladite substance d'intérêt dans l'échantillon.
